(19) Europäisches Patentamt
European Patent Office
Office européen des brevets

(11) Numéro de publication: **0 500 437 B1**

(12) # FASCICULE DE BREVET EUROPEEN

(45) Date de publication de fascicule du brevet: **26.07.95**

(51) Int. Cl.[6]: **C07C 233/20**, A61K 7/06, A61K 7/48, A61K 47/16, A61K 9/107

(21) Numéro de dépôt: **92400409.6**

(22) Date de dépôt: **17.02.92**

Le dossier contient des informations techniques présentées postérieurement au dépôt de la demande et ne figurant pas dans le présent fascicule.

(54) **Céramides, leur procédé de préparation et leurs applications en cosmétique et en dermopharmacie.**

(30) Priorité: **21.02.91 FR 9102091**

(43) Date de publication de la demande:
**26.08.92 Bulletin 92/35**

(45) Mention de la délivrance du brevet:
**26.07.95 Bulletin 95/30**

(84) Etats contractants désignés:
**AT BE CH DE DK ES FR GB GR IT LI NL PT SE**

(56) Documents cités:
**EP-A- 0 278 505**

**THE JOURNAL OF ORGANIC CHEMISTRY, vol. 46, no. 22, 23 octobre 1981, pages 4393-4398; P. TKACZUK et al.: "Useful syntheses of erythro- and threo-N-oleoyl-D-sphingosines (ceramides) and galactosylceramides (cerebrosides) from L-serine"**

**CHEMISTRY AND PHYSICS OF LIPIDS, vol. 13, no. 2, octobre 1974, pages 109-116;H. ALPES: "Synthesis of D,L-erythro-sphingomyelins"**

(73) Titulaire: **L'OREAL**
**14, rue Royale**
**F-75008 Paris (FR)**

(72) Inventeur: **Zysman, Alexandre**
**6, rue G.Eastman**
**F-75013 Paris (FR)**
Inventeur: **Vanlerberghe, Guy**
**40, rue du Général de Gaulle,**
**Villevaudé**
**F-77410 Claye-Souilly (FR)**
Inventeur: **Semeria, Didier**
**10, Allée des Lavandières**
**F-77181 Courtry (FR)**

(74) Mandataire: **Casalonga, Axel et al**
**BUREAU D.A. CASALONGA - JOSSE**
**Morassistrasse 8**
**D-80469 München (DE)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

**CHEMICAL ABSTRACTS**, vol. 107, no. 20, 16 novembre 1987, page 479, abrégé no. 183350j, Columbus, Ohio, US; & JP-A-62 120 308

**CHEMICAL ABSTRACTS**, vol. 106, no. 12, 23 mars 1987, page 348, abrégé no. 89979d, Columbus, Ohio, US; & JP-A-61 260 008

## Description

La présente invention a pour objet de nouveaux céramides, leur procédé de préparation ainsi que leur utilisation, notamment pour les traitements et les soins de la peau et des cheveux en cosmétique ou en dermopharmacie.

L'exposition de la peau au froid, au soleil, aux atmosphères à faible humidité relative, les traitements répétés avec des compositions de lavage ou encore le contact avec des solvants organiques, sont des facteurs qui entraînent, à des degrés divers, un déssèchement apparent. La peau apparaît plus sèche, moins souple et le relief cutané plus prononcé. Par ailleurs, les cheveux, qui sont soumis trop fréquemment à certains traitements capillaires, perdent leur aspect brillant et peuvent devenir rêches et cassants.

La demanderesse a donc recherché des composés qui permettent de prévenir ou du corriger ces phénomènes se traduisant par un déssèchement apparent et qui redonnent à la peau sa souplesse et aux cheveux leur brillance et leur douceur.

Pour résoudre ce problème, on a déjà proposé d'utiliser des céramides. On sait en effet que ces composés sont les éléments constitutifs prépondérants des lipides intercornéocytaires du stratum cornéum et participent au maintien de l'intégrité de la barrière cutanée. Ils représentent, selon DOWNING ("The Journal of Investigative Dermatology", vol. 88, n° 3, p. 2s-6s, 1987), environ 40% de la totalité de ces lipides.

Les céramides utilisés en cosmétique sont des extraits naturels issus notamment de la peau de porc, du cerveau de boeuf, de l'oeuf, des cellules du sang, des plantes etc... (demandes de brevets JA 86/260008 et JA 87/120308). De tels céramides ont également été proposés pour la protection des cheveux (EP 0278 505).

Il s'agit donc toujours de mélanges de teneur plus ou moins importante en céramides et dont la composition est difficile à contrôler. De plus, ces mélanges sont sujets à la contamination bactérienne. Leur conservation est très difficile à maîtriser. Lorsqu'ils sont d'origine animale, il y a en plus un risque de contamination par l'agent responsable de la BSE (encéphalopathie bovine spongiforme).

Pour résoudre ces problèmes, la demanderesse s'est donc tournée vers les céramides de synthèse.

C'est ainsi que la demanderesse a découvert de nouveaux composés dont la constitution peut être représentée par la formule suivante :

$$\begin{array}{l} R_1CHOH\underset{\displaystyle NHCOR_2}{\underset{|}{C}}HCH_2OH \end{array} \qquad (I)$$

dans laquelle:

. $R_1$ désigne un radical alcoyle ou alcényle en $C_{11}$ à $C_{21}$;

. $R_2$ désigne un radical hydrocarboné en $C_{11}$-$C_{19}$, linéaire et portant une ou plusieurs insaturations éthyléniques et notamment une ou deux, ou un mélange de radicaux hydrocarbonés en $C_{11}$-$C_{19}$, linéaires, saturés ou portant une ou plusieurs insaturations éthyléniques, et notamment une ou deux,dans lequel la proportion de radicaux saturés ne peut excéder 35 %,

ces composés étant sous forme de mélange racémique des diastéréoisomères érythro et thréo dans les proportions érythro: thréo de 85:15 à 60:40.

Les composés (I) selon l'invention, qui sont des cires de bas point de fusion, présentent donc un intérêt tout particulier pour les traitements et les soins de la peau et des cheveux en cosmétique ou en dermopharmacie en permettant de prévenir ou de corriger certains effets du dessèchement apparent.

Ces composés présentent pas ailleurs une faible agressivité vis-à-vis de la peau ou des muqueuses oculaires et une bonne tolérance vis-à-vis des membranes cellulaires comme celles des érythrocytes.

Les nouveaux composés de formule (I) ci-dessus présentent des propriétés émollientes et adoucissantes. Ils sont facilement solubilisés dans les phases grasses des préparations cosmétiques ou dermopharmaceutiques.

Les poils traités pas ces composés présentent un aspect brillant et une moins grande sensibilité à l'eau, due à l'apport de matière lipidique uniformément répartie sur les écailles du poil. Les propriétés mécaniques et de nervosité sont également améliorées.

Ces composés forment, en association avec d'autres lipides, des vésicules.

La présente invention a ainsi pour objet les nouveaux céramides de formule (I) définis ci-dessus, sous forme de mélange racémique des diastéréoisomères érythro et thréo dans les proportions de 85:15 à 60:40.

Les céramides de formule (I) ci-dessus résultent de l'acylation de la fonction amine d'une sphingosine ou d'une dihydrosphingosine par un acide activé de formule $R_2COA$ où $R_2$ a la signification indiquée ci-dessus et A peut prendre notamment les significations suivantes :

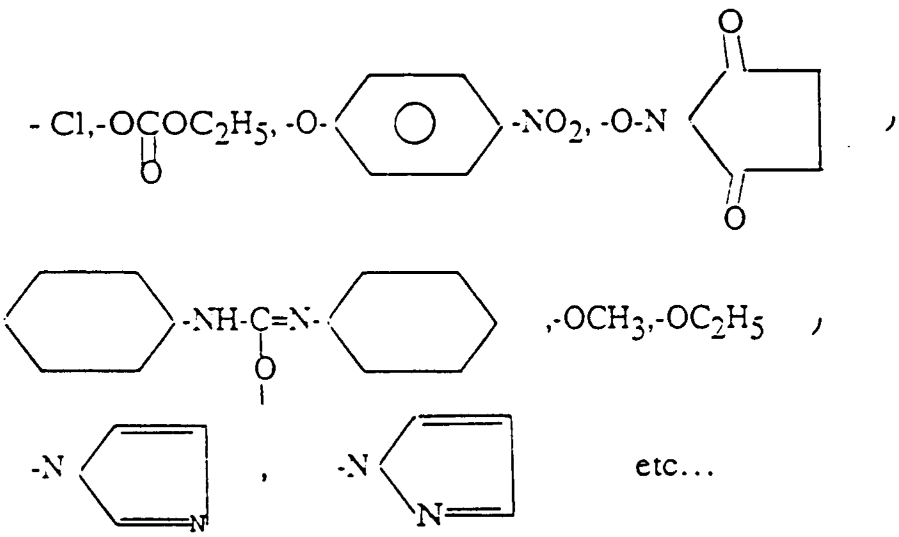

Dans la présente demande, on entendra par sphingosine ou dihydrosphingosine les composés D,L c'est-à-dire les mélanges racémiques des diastéréoisomères érythro et thréo.

Un autre objet de la présente invention est donc constitué par le procédé de préparation des composés de formule (I) qui peut être représenté par le schéma suivant:

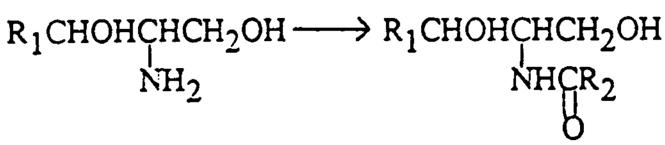

(II) (I)

$R_1$ et $R_2$ ayant les significations indiquées ci-dessus.

Les composés (I) sont obtenus par acylation des composés de formule (II) soit avec un chlorure d'acide, soit par un anhydride, soit par un ester de paranitrophénol, soit par un ester de succinimide, soit pas un ester de dicyclohexylcarbodiimide, soit par un ester d'alkyle inférieur, soit par un azolide et notamment un imidazolide ou un pyrazolide.

Les réactions d'acylation avec un ester d'alkyle inférieur se font à l'état anhydre. Elles sont notamment décrites pas E.F. JORDAN dans JAOCS p. 600-605 (1961).

Les autres réactions sont effectuées dans des solvants tels que le tétrahydrofuranne, la pyridine, le diméthylformamide, le dichlorométhane etc...

L'acylation par un ester de succinimide et de dicyclohexylcarbodiimide est décrite notamment par LAPIDOT dans J. Lipid Res. 8, 142-145 (1967).

L'acylation par un ester de paranitrophénol est décrite notamment par BODANSKY dans Nature n° 4459 p. 685 (1955).

L'acylation par un anhydride mixte est décrite par J.L. TORRES dans Tetrahedron vol. 43 n° 17, p. 4031-3 (1987).

Les acylations avec les azolides sont décrites par H.A. STAAB dans Angew, Chem. Internat. Edit. Vol. 1 n° 7 p. 357-367 (1962).

Les réactions d'acylation sont décrites en général par J. MARCH dans Advanced Organic Chemistry-Third Edition - JOHN WILEY & SONS-INC p. 370-377 (1985).

Pour la préparation du composé (I) de l'invention, on peut également utiliser le chlorhydrate du composé (II).

Les composés (II) sont des composés connus. Leur synthèse a été décrite en particulier par D. SHAPIRO dans "Chemistry of sphingolipids", HERMANN, Paris (1969).

Quand $R_1$ désigne un radical alcényle, les composés (II) sont des sphingosines dont la synthèse est décrite à la page 21 de "Chemistry of Sphingolipids".

Quand $R_1$ désigne un radical alcoyle, les composés (II) sont des dihydrosphingosines. Elles peuvent être préparées en particulier à partir de 2- acétamido-3-oxo-alcanoate de méthyle ou d'éthyle, comme décrit dans "Chemistry of Sphingolipids", page 32.

Les procédés de synthèse des sphingosines ou dihydrosphingosines décrits ci-dessus conduisent à des mélanges racémiques des diastéréoisomères érythro et thréo dans les proportions érythro-thréo de 85:15 à 60:40.

Les composés selon l'invention peuvent recevoir des applications diverses, notamment en tant que constituants cireux dans des compositions cosmétiques et dermopharmaceutiques. Ces composés possèdent en plus la propriété de former des vésicules en association avec d'autres lipides, lorsqu'ils sont dispersés dans l'eau.

La présente invention a donc pour objet l'utilisation des composés lipidiques de formule (I) en tant que constituants cireux dans des émulsions, des dispersions ou dans des lotions. Elle a également pour objet l'utilisation de ces composés, associés à d'autres lipides, pour la formation de sphérules lipidiques.

La présente invention a également pour objet des compositions à usage cosmétique ou dermopharmaceutique contenant un composé de formule (I).

Un autre objet de l'invention est constitué par un procédé de traitement cosmétique de la peau, des cheveux ou des poils consistant à appliquer sur ces derniers une quantité suffisante d'une telle composition contenant un composé de formule (I).

Les compositions selon l'invention peuvent se présenter sous forme d'émulsions (lait ou crème), de lotions hydroalcooliques, huileuses ou oléoalcooliques, de gels, de dispersions ou de bâtonnets solides, de sprays ou de mousses aérosol.

Selon l'invention, les composés de formule (I) représentent 0,05% à 20%, et de préférence 0, 1 à 10% du poids total de la composition.

Les compositions sont par exemple des lotions, des laits ou des crèmes émollients, des laits ou des crèmes pour les soins de la peau ou des cheveux, des crèmes, des lotions ou des laits démaquillants, des bases de fond de teint, des lotions, des laits ou des crèmes antisolaires, des lotions, des laits ou des crèmes de bronzage artificiel, des crèmes ou des mousses de rasage, des lotions après rasage, des shampooings ou des mascaras.

Ces compositions peuvent également se présenter sous la forme de bâtons pour les lèvres destinés soit à les colorer, soit à éviter les gerçures, ou de produits de maquillage pour les yeux ou de fards et fonds de teint pour le visage.

Lorsque les compositions selon l'invention se présentent sous forme d'émulsions du type eau-dans-l'huile ou huile-dans l'eau, la phase grasse est essentiellement constituée d'un mélange de composé de formule (I) avec au mois une huile, et éventuellement un autre corps gras.

La phase grasse des émulsions peut constituer 5 à 60% du poids total de l'émulsion.

La phase aqueuse desdites émulsions constitue de préférence 30 à 85% du poids total de l'émulsion.

La proportion de l'agent émulsionnant peut être comprise entre 1 et 20%, et de préférence entre 2 et 12% du poids total de l'émulsion.

Lorsque les compositions selon l'invention se présentent sous forme de lotions huileuses, oléoalcooliques ou hydroalcooliques, elles peuvent constituer, pas exemple, des lotions antisolaires contenant un filtre absorbant les rayons UV, des lotions adoucissantes pour la peau; les lotions huileuses peuvent en outre constituer des huiles moussantes contenant un tensio-actif oléosoluble, des huiles pour le bain, etc.

Parmi les principaux adjuvants pouvant être présents dans les compositions selon l'invention, on peut citer les corps gras tels que les huiles ou les cires minérales, animales ou végétales, les acides gras, les esters d'acides gras tels que les triglycérides d'acides gras ayant de 6 à 18 atomes de carbone, les alcools gras; les émulsionnants comme les alcools gras oxyéthylénés ou les alcoyléthers de polyglycérol; les solvants tels que les monoalcools ou polyalcools inférieurs contenant de 1 à 6 atomes de carbone ou encore l'eau.

Les mono- ou polyalcools plus particulièrement préférés sont choisis parmi l'éthanol, l'isopropanol, le propylèneglycol, le glycérol et le sorbitol.

A titre de corps gras, parmi les huiles minérales, on peut citer l'huile de vaseline; parmi les huiles animales, les huiles de baleine, de phoque, de menhaden, de foie de flétan, de morue, de thon, de tortue, de pied de boeuf, de pied de cheval, de pied de mouton, de vison, de loutre, de marmotte, etc.; parmi les huiles végétales, les huiles d'amande, de germe de blé, d'olive, de germe de maïs, de jojoba, de sésame, de tournesol, de palme, de noix, de karité, de shoréa, de macadamia, de pépins de cassis et similaires.

Parmi les esters d'acides gras, on peut utiliser des esters d'acides en $C_{12}$ à $C_{22}$ saturés ou insaturés et d'alcools inférieurs comme l'isopropanol ou le glycérol ou d'alcools gras en $C_8$ à $C_{22}$, linéaires ou ramifiés, saturés ou insaturés ou encore d'alcanediols-1,2 en $C_{10}$-$C_{22}$.

On peut également citer comme corps gras, la vaseline, la paraffine, la lanoline, la lanoline hydrogénée, le suif, la lanoline acétylée, les huiles de silicone.

Parmi les cires, on peut citer la cire de Sipol, la cire de lanoline, la cire d'abeille, la cire de Candelila, la cire microcristalline, la cire de Carnauba, le spermaceti, le beurre de cacao, le beurre de karité, les cires de silicone, les huiles hydrogénées concrètes à 25 °C, les sucroglycérides, les oléates, myristates, linoléates et stéarates de Ca, Mg et Al.

Parmi les alcools gras, on peut citer les alcools laurique, cétylique, myristique, stéarique, palmitique, oléique et les alcools de GUERBET comme le 2-octyldodécanol, le 2-décyltétradécanol ou le 2-hexyldécanol.

A titre d'émulsionnants, parmi les alcools gras polyoxyéthylénés, on peut citer les alcools laurique, cétylique, stéarylique et oléique comportant de 2 à 20 moles d'oxyde d'éthylène et parmi les alcoyléthers de polyglycérol, les alcools en $C_{12}$-$C_{18}$ comportant de 2 à 10 moles de glycérol.

Il peut aussi être utile d'utiliser des épaississants tels que les dérivés de cellulose, les dérivés d'acide polyacrylique, les gommes de guar ou de caroube ou la gomme de xanthane.

La composition selon l'invention peut également contenir des adjuvants habituellement utilisés en cosmétique ou en dermopharmacie et notamment des produits hydratants, des adoucissants, des produits pour le traitement d'affections cutanées, des filtres solaires, des germicides, des colorants, des conservateurs, des parfums et des propulseurs.

Lorsque les compositions selon l'invention sont des dispersions, il peut s'agir de dispersions de composés de formule (I) dans l'eau en présence de tensio-actif ou encore de dispersions aqueuses de sphérules lipidiques, constituées de couches moléculaires organisées enfermant une phase aqueuse encapsulée, ces couches étant constituées d'au mois un composé de formule (I), asscocié à au moims un autre composé lipidique.

On peut citer, à cet effet, comme composés lipidiques, les alcools et diols à longue chaîne, les stérols tels que le cholestérol, les phospholipides, les cholestéryl sulfate et phosphate, les amines à longue chaîne et leurs dérivés d'ammonium quaternaire, les dihydroxyalkylamines, les amines grasses polyoxyéthylénées, les esters d'aminoalcools à longue chaîne, leurs sels et dérivés d'ammonium quaternaire, les esters phosphoriques d'alcools gras tels que le dicétylphosphate acide ou son sel de sodium, les alkylsulfates tels que le cétysulfate de sodium, les acides gras sous forme de sels ou encore les lipides du type de ceux décrits dans les brevets français n° 2 315 991, 1 477 048 et 2 091 516 ou dans la demande de brevet iternational WO 83/01 571.

On peut par exemple utiliser comme autres lipides, des lipides comportant une chaîne lipophile longue contenant 12 à 30 atomes de carbone, saturée ou insaturée, ramifiée ou linéaire, par exemple une chaîne oléique, lanolique, tétradécylique, hexadécylique, isostéarylique, laurique ou alcoylphénylique. Le groupement hydrophile de ces lipides peut être un groupement ionique ou nonionique. A titre de grougements non-ioniques, on peut citer des groupements dérivés de polyéthylèneglycol. On peut aussi utiliser avantageusement comme lipides formant la phase lamellaire, des éthers de polyglycérol tels que ceux décrits dans les brevets français n° 1 477 048, 2 091 516, 2 465 780 et 2 482 128.

A titre de groupement ionique, on peut avantageusement utiliser un groupement dérivé d'un composé amphotère, anionique ou cationique.

D'autres lipides décrits dans la demande de brevet international WO 83/01 571 comme pouvant être utilisés pour la formation de vésicules sont les glycolipides comme le lactosylcéramide, le galactocérébroside, les gangliosides et le trihexosylcéramide, ainsi que les phospholipides tels que le phosphatidylglycérol et le phosphatidylinositol.

La présente invention a donc également pour objet une dispersion de sphérules lipidiques constituées de couches moléculaires organisées de composé(s) de formule (I) et de lipide défini ci-dessus renfermant une phase aqueuse à encapsuler.

La phase continue de la dispersion qui entoure les sphérules est de phase aqueuse.

Les sphérules en dispersion ont un diamètre compris entre 0,05µm et 5µm.

La phase aqueuse encapsulée dans les sphérules peut être de l'eau ou une solution aqueuse du substance active et est dans ce cas de préférence isoosmotique par rapport à la phase continue de la dispersion.

Les sphérules peuvent être obtenues en particulier suivant le procédé décrit dans le brevet français 2 315 991 de la demanderesse selon lequel on prépare une dispersion de sphérules constituées de couches moléculaires organisées refermant une phase aqueuse à encapsuler, en mettant en contact, d'une par un

ou plusieurs composé(s) lipidique(s) de formule (I) associé(s) à un ou plusieurs lipide(s) défini(s) ci-dessus, et d'autre part la phase aqueuse à encapsuler dans les sphérules, en agitant pour assurer le mélange et obtenu une phase lamellaire, en ajoutant ensuite un liquide de dispersion en quantité supérieure à la quantité de phase lamellaire obtenue et en secouant énergiquement pendant une durée allant de 15 miutes à 3 heures environ.

Le rapport pondéral entre la phase aqueuse à encapsuler et le(s) composé(s) de formule (I) associé aux lipides formant la phase lamellaire, est de préférence compris entre 0,1 et 20.

Le rapport pondéral de la phase aqueuse de dispersion que l'on ajoute à la phase lamellaire que l'on disperse, est de préférence compris entre 2 et 100, la phase de dispersion et la phase aqueuse à encapsuler étant de préférence isoosmotiques.

L'agitation est réalisée au moyen d'un agitateur à secousses. Le procédé est de préférence mis en oeuvre à une température comprise entre 30° et 120°C.

Un autre procédé de préparation peut consister à utiliser le procédé dénommé REV (reverse-phase evaporation vesicle) ou évaporation en phase inverse décrit dans Proc. Natl. Acad. Sci. USA., Vol. 75, n°9, pages 4194-4198 (1978), par SZOKA et PAPAHADJOPOULOS.

On peut également mettre en oeuvre le procédé qui comprend la succession d'étapes consistant à dissoudre au moins un lipide dans au moins un solvant organique non miscible à l'eau; ajouter la phase organique ainsi obtenue à une phase aqueuse; former une dispersion des deux phases sous forte agitation, la taille des vésicules pouvant être réglée en faisant varer la vitesse d'agitation au cours de ce mélange des phases; conduire l'évaporation du (ou des) solvant(s) sous forte agitation; et, le cas échéant, concentrer la dispersion.

Les substances actives peuvent être des substances ayant un intérêt pharmaceutique, alimentaire ou des substances ayant une activité cosmétique. Lorsqu'elles sont hydrosolubles, elles sont dans la phase aqueuse encapsulée à l'intérieur des vésicules.

Les substances hydrosolubles ayant une activité cosmétique et/ou pharmaceutique peuvent être des produits destinés aux soins ou aux traitements de la peau et du cheveu tels que par exemple des humectants comme la glycérine, le sorbitol, le pentaérythritol, l'acide pyrrolidone carboxylique et ses sels; des agents de brunissage artificiel tels que la dihydroxyacétone, l'érythrulose, le glycéraldéhyde, les $\gamma$-dialdéhydes tels que l'aldéhyde tartrique, ces composés étant éventuellement associés à des colorants; des filtres solaires hydrosolubles; des antiperspirants, des déodorants, des astringents, des produits rafraîchissants, toniques, cicatrisants, kératolytiques, dépilatoires, des eaux parfumées; des extraits de tissus végétaux, tels que les polysaccharides; des colorants hydrosolubles; les agents antipelliculaires; des agents antiséborrhéiques, des oxydants tels que des agents de décoloration comme l'eau oxygénée; des réducteurs tels que l'acide thioglycolique et ses sels.

On peut citer également les vitamines, les hormones, les enzymes telles que la superoxyde dismutase, les vaccins, les anti-inflammatoires tels que l'hydrocortisone, les antibiotiques, les bactéricides, les agents cytotoxiques ou anti-tumoraux.

Lorsque les substances actives sont liposolubles, elles se trouvent incorporées dans les feuillets des vésicules. Elles peuvent être choisies dans le groupe formé par les filtres solaires liposolubles, les substances destinées à améliorer l'état des peaux sèches ou séniles, les tocophérols, les vitamines E, F ou A et leurs esters, l'acide rétinioïque, les antioxydants, les acides gras essentiels, l'acide glycyrrhétinique, les kératolytiques et les caroténoïdes.

On peut égalenent ajouter à la phase aqueuse des dispersions de sphérules selon l'invention une phase liquide L non miscible à l'eau. En particulier, la composition selon l'invention peut contenir de 2 à 70% en poids de phase liquide L, non miscible à l'eau, par rapport au poids total de la composition, la proportion pondérale relative de(s) lipide(s) constitué(s) de vésicules par rapport à la phase liquide dispersée L étant comprise entre 0,02/1 et 10/1.

Le(s) constituant(s) de la phase liquide L dispersée dans la phase aqueuse D, peut (peuvent) être choisi(s) dans le groupe formé par les huiles, telles que les esters d'acides gras et de polyols, et les esters d'acide gras et d'alcools ramfiés de formule $R^7$-$COOR^8$, fomule dans laquelle $R^7$ représente le reste d'un acide gras supérieur comportant de 7 à 19 atomes de carbone et $R^8$ représente une chaîne hydrocarbonée ramifiée contenant de 3 à 20 atomes de carbone; les hydrocarbures, tels que l'hexadécane, l'huile de paraffine, le perhydrosqualène; les hydrocarbures halogénés, tels que le perfluorodécahydronaphtalène; la perfluorotributylamine; les polysiloxanes; les esters d'acides organiques, les éthers et polyéthers. La phase liquide L peut renfermer au moins un parfum et/ou au mois une substance active liposoluble. De telles substances liposolubles peuvent être constituées par les filtres solaires liposolubles, les substances destinées à améliorer l'état des peaux sèches ou séniles, les tocophérols, les vitamines E ou F, la vitamine A et ses esters, l'acide rétinoïque, les antioxydants, les acides gras essentiels, l'acide glycyrrhétinique, les

agents kératolytiques et les caroténoïdes.

On peut également ajouter aux dispersions de sphérules selon l'invention divers adjuvants tels que des opacifiants, des gélifiants, des arômes, des parfums ou des colorants.

Les dispersions de sphérules lipidiques selon l'invention présentent l'intérêt de véhiculer des substances actives qui se trouvent aisi masquées et protégées vis-à-vis des différents agents d'altération: oxydants et plus généralement composés réactifs vis-à-vis des substances actives encapsulées. La pénétration et la fixation des substances actives peuvent être modulées pas la variation de la taille des sphérules et de leur charge électrique. L'action de ces substances actives peut également être ainsi différée (effet retard). Enfin, il est possible d'obtenir grâce à l'utilisation des lipides (I) selon l'invention, et de substances actives combinées une action bénéfique spécifique de la substance active utilisée et en même temps assouplissante, particulièrement intéressante dans le cas de traitement de la peau.

La présente invention a donc également pour objet l'utilisation en cosmétique d'une dispersion aqueuse de splérules constituée de couches moléculaires organisées de composés lipidiques (I) associés à d'autres lipides renfermant une phase aqueuse à encapsuler, en particulier pour le traitement de la peau.

L'invention a également pour objet l'utilisation d'une telle dispersion de sphérules lipidiques en dermopharmacie ou dans l'industrie alimentaire.

La présente invention sera mieux illustrée par les exemples non limitatifs suivants.

EXEMPLE 1

Préparation du 2-oléoylamino- 1,3-octadécanediol

1ère étape

Préparation du composé (II) avec: $R_1 = C_{15}H_{31}$ ; chlorhydrate du 2-amino-1,3-octadécanediol (mélange érythrothréo)

Le 2-acétamido-3-oxo octadécanoate de méthyle (100 g, soit 0,27 M) est mis en suspension dans 1 litre d'éthanol absolu. La température du milieu réactionnel est amenée en dessous de 0 °C. A cette température, on ajoute en trois fois 30,7 g (0,8 M) de borohydrure de sodium et on maintient l'agitation à cette température pendant 3 heures. Le milieu réactionnel est alors porté à reflux du solvant pendant 3 heures. Après refroidissement à la température ordinaire, on ajoute 140 $cm^3$ d'acide chlorhydrique concentré et on porte à nouveau le milieu réactionnel à reflux pendant 3 heures. Ce milieu est filtré encore chaud sur un verre fritté. Le filtrat est concentré à sec sous pression réduite.

Le solide obtenu est recristallisé dans 300 $cm^3$ de mélange de solvant heptane: acétate d'éthyle = 90 : 10. On isole 88 g d'un solide blanc dont l'indice d'acide mesuré dans l'éthanol par une solution de soude N/10 est de 2,99 meq/g.

Le spectre RMN$^{13}$C de ce solide est conforme à la structure attendue

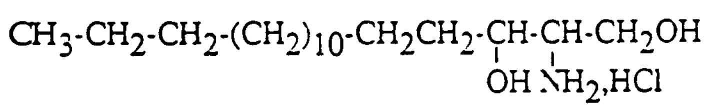

Il s'agit bien du chlorhydrate de dihydrosphingosine sous forme de mélange racémique D,L érythrothréo.

2ème étape

Préparation du composé (I) dans lequel -$COR_2$ = oléoyle

Matières Premières

| | |
|---|---|
| - Chlorhydrate de dihydrosphingosine préparé dans la première étape | 100 g |
| - Chlorure d'oléoyle (1) | 84,7 g |
| - Triéthylamine | 60 g |
| - Tétrahydrofurane | 400 ml |
| - Isopropanol | 150 ml |
| - Résine AMBERLITE IRN 150 | 160 g |
| - Acétate d'éthyle | 600 ml |

(1) Commercialisé par BASF avec un titre en chlorure d'acide de 98%.
La répartition des principales chaînes est la suivante :

| | |
|---|---|
| $C_{14}$ : 4,5% | $C_{1S}$=1 : 65% |
| $C_{16}$ : 8,5% | $C_{1S}$=2 : 4,8% |
| $C_{18}$ : 3,4% | $C_{18}$=3 : 0,5% |

Mode Opératoire

Dans un réacteur sous azote, on introduit 400 ml de tétrahydrofurane et 100 g de chlorhydrate de dihydrosphigosine (0,296 mole) que l'on disperse par chauffage à 45 °C; on ajoute alors 31 g de triéthylamine (0,307 mole), ce qui provoque un trouble du milieu réactionnel et une augmentation de la température à 48 °C; le pH "apparent" est alors de 8,3±0,1. On laisse revenir à 30 °C±1 °C.

Par une ampoule à introduction, on commence alors à introduire le chlorure d'oléoyle jusqu'à amener le pH "apparent" jusqu'à 6,7 et on le maintient à cette valeur en ajoutant simultanément en 1 heure, le reste du chlorure d'oléoyle et 29 g de triéthylamine (0,287 mole); au cours de ces additions, la température s'élève à 33 °C et est maintenue à cette valeur. On poursuit alors la réaction pendant deux heures et demie supplémentaires.

A l'issue de la réaction, le mélange réactiorinel est lavé trois fois par 200 ml d'eau à 50-60 °C, débarrassé du tétrahydrofurane par distillation sous pression réduite, et repris par 200 ml d'isopropanol à 50 °C. La solution trouble obtenue est agitée pendant une heure en présence de 180 g de résine AMBERLITE IRN 150 puis filtrée sur verre fritté n° 4 préchauffé; après lavage de la résine pas 50 ml d'isopropanol à 60 °C, la solution limpide jaune est versée lentement dans 600 ml d'acétate d'éthyle sous vive agitation. Le précipité obtenu est laissé au repos 15 heures à +4 °C puis essoré sur verre fritté n° 3. On obtient après séchage sous pression réduite à 40-45 °C, 64 g de produit blanc, soit un rendement de 40%.

ANALYSES

- F = 76 °
- Spectre IR conforme

- Spectre RMN 1H conforme

- Analyse élémentaire :

| | C% | H% | N% | O% |
|---|---|---|---|---|
| Calculé | 76,40 | 12,65 | 2,48 | 8,48 |
| Trouvé | 76,55 | 12,65 | 2,49 | 8,63 |

Rapport érythro : thréo = 75:25

Rapport érythro : thréo = 75:25

EXEMPLE 2

Préparation du 2-oléoylamino-1,3-octadécanediol

Dans un ballon, sous atmosphère inerte, on solubilise 3 ml de chloroformiate d'éthyle (31 mmoles) dans 5 ml de tétrahydrofurane; cette solution étant refroidie à -15 ° C, on additionne goutte à goutte 11,3 g d'oléate de triéthylamine (29,5 mmoles) préalablement solubilisés dans 10 ml de tétrahydrofurane.

Après 2 heures d'agitation à température ambiante, le milieu réactionnel est versé lentement, sous atmosphère inerte, dans 8,9 g de (D,L) 2-amino-1,3-octadécanediol préparé à la 1ère étape de l'exemple 1 (29,5 mmoles) solubilisés dans 40 ml de tétrahydrofurane, à 30 ° C.

Après 1 heure d'agitation, à 30 ° C, sous atmosphère inerte, (chromatographie sur couche mince de gel de silice, éluant chlorure de méthylène/méthano/ammoniaque 15/3,5/0,6), le milieu réactionnel est lavé à l'eau, puis évaporé à sec, sous vide. Le produit brut obtenu est chromatographié sur colonne de silice (éluant : acétate d'éthyle/heptane 5/3) et après évaporation du solvant d'élution, sous vide, on obtient 12,5 g de cristaux blancs (rendement 75%).

Les spectres RMN $^{13}$ C et IR sont conformes à la structure attendue.

Analyse centésimale : $C_{36}H_{71}NO_3$

| | C% | H% | N% | O% |
|---|---|---|---|---|
| Calculé | 76,40 | 12,65 | 2,48 | 8,48 |
| Trouvé | 76,47 | 12,75 | 2,60 | 8,72 |

Rapport Erythro : Thréo = 84:16

EXEMPLE 3

Préparation du 2-linoléoylamino-1,3-octadécanediol

Selon le même mode opératoire que celui décrit à l'exemple 2 et en utilisant 11,3 g de linoléate de triéthylamine (29,5 mmoles), on obtient 11,6 g de cristaux blancs (rendement 70%).

Les spectres RMN$^{13}$ C et I R sont conformes à la structure attendue.

Analyse centésimale : $C_{36}H_{69}NO_3$

| | C% | H% | N% | O% |
|---|---|---|---|---|
| Calculé | 76,67 | 12,33 | 2,48 | 8,51 |
| Trouvé | 76,74 | 12,38 | 2,49 | 8,64 |

Rapport Erythro : Thréo = 74:26

EXEMPLE 4

Préparation du 2-linoléoylamino-1,3-octadécanediol

Selon le même mode opératoire que celui décrit à l'exemple 2 et en utilisant 11,3 g de linoléate de triéthylamine *(29,5 mmoles), on obtient 11,6 g de cristaux blancs (rendement 49%).

Le spectre RMN $^{13}C$ est conforme à la structure attendue.

Le spectre de masse est en accord avec la structure attendue.

Rapport Erythro: Thréo = 66:34

EXEMPLES DE FORMULATION

EXEMPLE 1

Dipersion vésiculaire

La préparation est réalisée à l'aide des parties suivantes :

Partie A

. Céramide suivant exemple 1 : 0,90 g
. Cholestérylsulfate de sodium : 0,60 g

Partie B

. Eau: 1,50 g

Partie C

. Tampons phosphate 0,1 M, pH = 6,93 : 27,10 g

Etape 1

Les constituants de la partie A sont introduits dans un ballon de 100 ml puis dissous dans un mélange contenant 5 ml de chloroforme et 20 ml de méthanol. Le solvant est ensuite évaporé à 40 °C sous pression réduite (pression finale: environ $5x10^2$ Pa), à l'aide d'un évaporateur rotatif.

Etape 2

Le film lipidique formé à l'issue de l'étape 1 est prélevé et placé dans un flacon de 15 ml. On ajoute aux lipides la partie B. Le mélange obtenu subit le cycle suivant: homogénéisation à l'aide d'une spatule-chauffage en étuve à 75 °C pendant une durée de 15 minutes; retour à temperature ambiante par refroidissement spontané à l'air. Le cycle est reproduit en chauffant une fois 15 minutes puis une autre fois en chauffant 5 minutes.

*A partir d'une vitamine F du commerce dont la composition est la suivante:

$C_{14}$: 0,1 %
$C_{16}$: 6,5%
$C_{18}^{=1}$: 15,5%
$C_{18}^{=2}$: 72,4%
$C_{18}^{=3}$: 3,4%

Etape 3

A la phase lamellaire issue de l'étape 2, on ajoute la partie C. Le mélange ainsi obtenu est agité par secouage pendant 1heure à 70 °C.

On obtient ainsi une dispersion blanche de vésicules. La fraction volumique des vésicules est égale à 18% et leur taux d'encapsulation est égal à 2, 6µl de milieu aqueux par mg de mélange lipidique

EXEMPLE 2

Dispersion vésiculaire

La préparation est réalisée à l'aide des parties suivantes:

Partie A

. Composé non-ionique I : 0,56 g

I : formule générale

$$C_{16}H_{33}O\text{-}[\text{-}C_3H_5\text{-}(OH)\text{-}O\text{-}]_n\text{-}H$$

où : $-C_3H_5(OH)-O$ est représenté par les structures suivantes prises en mélange ou séparément:

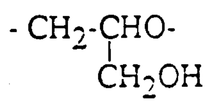

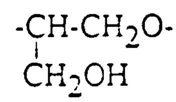

où n est une valeur statistique moyenne égale à 3

. cholestérol : 0,71 g

. dihexadécylphosphate de sodium : 0,08 g

. céramide suivant l'exemple 1 : 0,15 g

Partie B

. Solution contenant 0,02% d'azoture de sodium dans l'eau : 28,50g

Etape 1

Les constituants de la partie A sont introduits dans un ballon de 100 ml puis dissous dans un mélange contenant 10 ml de dichlorométhane et 4 ml de méthanol.

Le solvant est ensuite évaporé à 41 °C sous pression réduite, par paliers successifs depuis la pression ambiante jusqu'à environ $5x10^2$ Pa, à l'aide d'un évaporateur rotatif.

Etape 2

Au film lipidique formé à l'issue de l'étape 1, on ajoute la partie B. Le mélange ainsi obtenu est agité par secouage pendant 2 heures à 70 °C.

On obtient ainsi une dispersion blanche de vésicules.

La fraction volumique des vésicules est égale à 53% et leur taux d'encapsulation est égal à 9,4µl de milieu aqueux par mg de mélange lipidique.

Etape 3

La dispersion obtenue à l'issue de l'étape 2 est portée à la température de 30 °C, et traitée pendant 2 minutes à l'aide d'un homogénéisateur à ultrasons (Sonifier B 3.0 vendu par la Société BRANSON SONIC POWER Co), équipé d'une microsonde.

Conditions de réglage :

- cycle de travail : 50 %

- réglage de puissance : position 5

On obtient ainsi une dispersion blanchâtre de vésicules.

La fraction volumique des vésicules est égale à 19% et leur taux d'encapsulation est égal à 2,9µl de milieu aqueux par mg de mélange lipidique.

La taille moyenne des vésicules mesurée après 1 jour de conservation à température ambiante est égale à (182±4)nm; après une semaine: (179±3)nm; après 1 mois: (177±4) nm.

EXEMPLE 3

| Soin pour la peau : Crème H/E | | (% en poids) |
|---|---|---|
| - Stéarate de glycérol | | 2 |
| - Monostéarate de sorbitan à 20 moles d'oxyde d'éthylène | | 1 |
| - Alcool cétylique | | 0,5 |
| - Acide stéarique | | 1,4 |
| - Triéthanolamine | | 0,7 |
| - Acide polyacrylique réticulé commercialisé sous le nom de "CARBOPOL 940" | | 0,4 |
| - Fraction liquide de graisse de karité | | 12 |
| - Perhydrosqualène de synthèse | | 12 |
| - Antioxydants | | 0,05 |
| - Céramide suivant l'exemple 1 ou 2 | | 0,2 |
| - Parfum | | 0,5 |
| - Eau + conservateurs | qsp | 100 |

EXEMPLE 4

| Soin pour la peau : Crème E/H | | (% en poids) |
|---|---|---|
| - Monoisostéarate de sorbitan | | 5 |
| - Cire microcristalline | | 1 |
| - Huile de vaseline | | 10 |
| - Huile de germes de maïs | | 4 |
| - Esters d'acides gras en $C_8$-$C_{18}$ et d'alcools gras en $C_{12}$-$C_{18}$ | | 1 |
| - Octyldodécanol | | 4,9 |
| - Céramide suivant l'exemple 1 ou 2 | | 0,35 |
| - Gel de montmorillonite modifiée et d'huile neutre (triglycérides d'acides caprylique et caprique) | | 5 |
| - Propylène glycol | | 3 |
| - Antioxydants | | 0,1 |
| - Eau + conservateurs | qsp | 100 |

EXEMPLE 5

| Soin pour la peau : lait corporel | | (% en poids) |
|---|---|---|
| - Stéarate de glycérol | | 2 |
| - Monostéarate de sorbitan à 20 moles d'oxyde d'éthylène | | 1 |
| - Acide stéarique | | 1,4 |
| - Triéthanolamine | | 0,7 |
| - Acide polyacrylique réticulé commercialisé sous le nom de "CARBOPOL 940" | | 0,2 |
| - Huile d'amandes douces | | 3 |
| - Huile de vaseline | | 8 |
| - Antioxydants | 0,05 | |
| - Céramide suivant l'exemple 1 ou 2 | | 0,3 |
| - Eau + conservateurs | qsp | 100 |

EXEMPLE 6

| Rouge à lèvres | | (% en poids) |
|---|---|---|
| - Huile de sésame | | 25 |
| - Lanoline | | 20 |
| - Cire de Carnauba | | 20 |
| - Céramide suivant l'exemple 1 ou 2 | | 5 |
| - Pigments | | 10 |
| - Huile de vaseline | | 100 |

EXEMPLE 7

| Rouge à lèvres | | (% en poids) |
|---|---|---|
| - Huile de jojoba | | 20 |
| - Lanoline | | 25 |
| - Cire microcristalline | | 20 |
| - Céramide suivant l'exemple 1 ou 2 | | 5 |
| - Pigments | | 10 |
| - Triglycérides d'acides caprique/caprylique vendus sous la dénomination "MIGLYOL 812" | qsp | 100 |

EXEMPLE 8

Dispersion vésiculaire

Dans un ballon rond de 100ml, on dissout dans 10ml d'un mélange solvant (chloroforme-méthanol dans le rapport 2/1), les produits suivants :

| | |
|---|---|
| - Céramide de l'exemple | 1 120 mg |
| - Cholestérol | 75 mg |
| - Acide palmitique | 75 mg |
| - Cholestérylsulfate de sodiun | 30 mg |

On évapore le solvant à l'aide d'un évaporateur rotatif et on élimine les dernières traces de solvant par passage à la pompe à palettes pendant 1 heure.

On met au contact l'association de lipides obtenue avec 10 g d'eau déminéralisée dans lauelle on a dissous 17,7 mg de dihydrogénophosphate de potassium et 75,5 mg de monohydrogénophosphate disodique.

On homogénéise le mélange à l'aide d'une secoueuse pendant 2 heures à la température de 90 °C, puis on revient progressivement à la température ambiante.

On obtient ainsi une dispersion de vésicules lipidiques dont la taille moyenne est de 0,2 micron.

EXEMPLE 9

Shampooing

On prépare un shampooing limpide de composition suivante:

| | | | |
|---|---|---|---|
| - Céramide de l'exemple 1 | | | 0,5 g |
| - Chlorure de sodium | | | 5 g |
| - Laurylsulfate de triéthanolamine à 40% MA (matière active) | | | 20 g MA |
| - Triéthanolamine | qs | pH | 7 |
| - Eau | qsp | | 100 g |

EXEMPLE 10

Lotion pour cheveux

On prépare une lotion non rincée de composition suivante:

| | |
|---|---|
| - Céramide de l'exemple 1 | 0,4 g |
| - Décaméthylcyclopentasiloxane vendu sous la dénomination "SILBIONE 70045 V 5" par la Société RHONE POULENC | 20 g |
| - Octaméthylcyclotétrasiloxane vendu sous la dénomination "SILBIONE 70045 V 2" par la Société RHONE POULENC | 20 g |
| - Hexaméthyldisiloxane vendu sous la dénomination "HUILE AK 0,65" par la Société WACKER | 19.6 g |
| - Alcool éthylique | 100 g |

EXEMPLE 11

Soin pour cheveux

On prépare un soin non rincé pour cheveux abîmés de composition suivant :

| | |
|---|---|
| - Décaméthylcyclopentasiloxane | 12,5 g |
| - Octaméthylcyclotétrasiloxane | 12,5 g |
| - Céramide de l'exemple 1 | 0,1 g |
| - Mélange d'un polydiméthylsiloxane (13%) hydroxylé en bout de chaîne et de cyclométhicone (87%) vendu sous la dénomination "Q2 - 1401" par la Société DOW CORNING | 65 g |
| - Benzoate d'alcools gras en $C_{12}$-$C_{15}$ vendu sous la dénomination "FINSOLV TN" | 4,95 g |
| - Alcool éthylique | 100 g |

Cette composition facilite le coiffage, apporte de la brillance et du maintien à la coifure.

EXEMPLE 12

Mousse restructurante

| | | |
|---|---|---|
| - Céramide de l'exemple 1 | | 1 g |
| - Monolaurate de sorbitane oxyéthyléné à 20 moles d'oxyde d'éthylène vendu sous la dénomination "TWEEN 20" par la Société ICI | | 5 g |
| - Conservateurs | | |
| - Eau | qsp | 100 g |
| - pH = 5,8 | | |

Conditonnement en bombe pressurisée

| | |
|---|---|
| - Actif | 92 g |
| - Propulseur hydrocarboné vendu sous le nom commercial "AEROGAZ 3,2N par la Société ELF Aquitaine | 8 g |

Appliquée en non rincé sur des cheveux décolorés après un shampooing, la composition améliore le dêmelage des cheveux mouillés et apporte un toucher lisse et uniforme.

EXEMPLE 13

Après shampooing

| | |
|---|---|
| - Mélange d'alcool cétylstéarylique et d'alcool cétylstéarylique oxyéthyléné à 33 moles d'oxyde d'éthylène vendu sous la dénomination "SINNOWAX AO" par la Société HENKEL | 2 g |
| - Alcool cétylique | 1 g |
| - Alcool stéarylique | 1 g |
| - Hydroxyéthylcellulose vendue sous la dénomination "NATROSOL 250 HR" par la Société HERCULES | 1 g |
| - Céramide de l'exemple 1 | 0,5 g |
| - Conservateurs | |
| - Eau qsp | 100 g |
| - pH = 6 | |

Appliquée sur cheveux décolorés après un shampooing, la composition apporte un toucher du cheveu plus durci et plus uniforme.

EXEMPLE 14

Mascara

| | | |
|---|---|---|
| - Stéarate de triéthanolamine | | 15 g |
| - Paraffine | | 5 g |
| - Cire d'abeilles | | 4 g |
| - Cire de Carnauba | | 4 g |
| - Céramide de l'exemple 1 | | 0,5 g |
| - Gomme arabique | | 0,8 g |
| - Oxyde de fer noir | | 5 g |
| - Parahydroxybenzoate de propyle | | 0,08 g |
| - Parahydroxybenzoate de méthyle | | 0,24 g |
| - Eau | qsp | 100 g |

MODE OPERATOIRE

1 - On fait fondre les cires (80 °C) et on y incolore le pigment.
2- On fait chauffer la phase aqueuse contenant la gomme et les conservateurs.
3 - On amène les deux phases à 75 °C et on ajoute le céramide dans la phase cireuse.
4 - On mélange ces deux phases afin de réaliser l'émulsion.

**Revendications**

**Revendications pour les Etats contractants suivants : AT, BE, CH, DE, DK, FR, GB, IT, LI, NL, PT, SE**

**1.** Céramide synthétique comprenant un composé de formule :

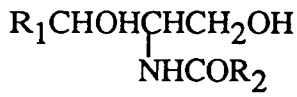

(I)

dans laquelle :

. $R_1$ désigne un radical alcoyle ou alcényle en $C_{11}$ à $C_{21}$;

. $R_2$ désigne un radical hydrocarboné en $C_{11}$-$C_{19}$, linéaire et portant une ou plusieurs insaturations éthyléniques, et notamment une ou deux, ou un mélange de composés de formule (I) et de formule :

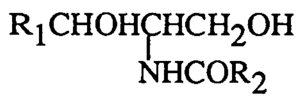

(Ia)

dans laquelle $R_1$ est défini comme précédemment et $R_2$ est un radical hydrocarboné linéaire saturé en $C_{11}$-$C_{19}$, la proportion de composés de formule (Ia) dans le mélange n'excédant pas 35%,

ce céramide étant sous la forme d'un mélange racémique des diastéréoisomères érythro et thréo dans des proportions érythro:thréo de 85:15 à 60:40.

**2.** Céramide synthétique selon la revendication 1, caractérisé par le fait que $R_1$ est un radical en $C_{15}$ dérivé de sphingosine, ou dihydrosphingosine.

**3.** Céramide synthétique selon la revendication 1 ou 2, caractérisé par le fait que $R_2$ est un radical dérivé de l'acide oléique, ou linoléique.

**4.** Céramide synthétique selon l'une quelconque des revendications 1 à 3, caractérisé par le fait qu'il est choisi parmi le 2-oléoylamino-1,3-octadécanediol et le 2-linoléoylamino-1,3-octadécanediol.

**5.** Procédé de préparation d'un céramide selon l'une quelconque des revendications 1 à 4, caractérisé par le fait qu'il consiste à acyler la fonction amine d'une sphingosine, ou d'une dihydrosphingosine de formule (II) suivante

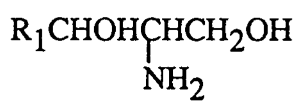

(II)

dans laquelle $R_1$ désigne un radical alcoyle ou alcényle en $C_{11}$ à $C_{21}$, ce composé étant sous forme de mélange racémique des diastéréoisomères érythro et thréo dans les proportions érythro:thréo de 85:15 à 60:40,

par un chlorure d'acide, par un anhydride, par un ester de paranitrophénol, par un ester de succinimide, par un ester de dicyclohexylcarbodiimide, par un ester d'alkyle inférieur ou par un azolide, en milieu anhydre ou dans des solvants tels que le tétrahydrofuranne, la pyridine, le diméthylformamide et le dichlorométhane.

**6.** Procédé de préparation d'un céramide selon la revendication 5, caractérisé par le fait que l'anhydride est un anhydride mixte de formule $R_2COOCOOC_2H_5$, $R_2$ ayant la signification indiquée dans la revendication 1, l'ester d'alkyle inférieur est un ester de méthyle ou d'éthyle et l'azolide est un imidazolide ou un pyrazolide.

**7.** Composition à usage cosmétique ou dermopharmaceutique, caractérisée par le fait qu'elle contient 0,05 à 20% en poids, de préférence 0,1 à 10% en poids, d'au moins un céramide selon l'une quelconque des revendications 1 à 4, en présence d'un adjuvant choisi parmi les corps gras, les solvants, l'eau, les épaississants, les émulsionnants, les produits hydratants, les adoucissants, les filtres solaires, les germicides, les colorants, les conservateurs, les parfums, les propulseurs et les tensio-actifs.

**8.** Composition selon la revendication 7, caractérisée par le fait qu'elle se présente sous forme d'émulsion dont la phase grasse, représentant 5 à 60% du pois total de l'émulsion, est essentiellement constituée d'un mélange du céramide avec au moins une huile, la phase aqueuse constituant 30 à 85% du poids total de l'émulsion, l'agent émulsionnant étant présent à raison de 1 à 20% en poids, et de préférence 2 à 12% en poids par rapport au poids total de l'émulsion.

**9.** Composition selon la revendication 7, caractérisée par le fait qu'elle se présente sous forme de lotion huileuse, oléoalcoolique ou hydroalcoolique, sous forme de gel, de dispersion, de bâtonnets solides, de spray ou de mousse aérosol.

**10.** Composition selon la revendication 7, caractérisée par le fait qu'elle se présente sous forme d'une dispersion aqueuse de sphérules lipidiques, constituées de couches moléculaires organisées renfermant une phase aqueuse encapsulée, ces couches étant constituées du ou des céramides associés à au moins un autre composé lipidique.

**11.** Composition sous forme de dispersion aqueuse de sphérules lipidiques selon la revendication 10, caractérisée par le fait que l'autre composé lipidique est choisi parmi les alcools et diols à longue chaîne, les stérols, les phospholipides, les glycolipides, les cholestéryl sulfate et phosphate, les amines à longue chaîne et leurs dérivés d'ammonium quaternaire, les dihydroxyalkylamines, les amines grasses polyoxyéthylénées, les esters d'aminoalcools à longue chaîne, leurs sels et dérivés d'ammonium quaternaire, les esters phosphoriques d'alcools gras, les alkylsulfates et les acides gras sous forme de sels.

**12.** Composition sous forme de dispersion aqueuse de sphérules lipidiques selon la revendication 10 ou 11, caractérisée par le fait que les sphérules ont un diamètre compris entre 0,05$\mu$m et 5$\mu$m.

**13.** Composition sous forme de dispersion aqueuse de sphérules lipidiques selon la revendication 10 ou 11, caractérisée par le fait que la phase aqueuse encapsulée dans les sphérules est de l'eau ou une solution aqueuse de substance active.

**14.** Composition sous forme de dispersion aqueuse de sphérules lipidiques selon l'une quelconque des revendications 10 à 13, caractérisée par le fait que la phase aqueuse encapsulée dans les sphérules contient au moins une substance active hydrosoluble cosmétique et/ou pharmaceutique choisie parmi les humectants, les agents de brunissage artificiel éventuellement associés à des colorants, les filtres solaires hydrosolubles, les antiperspirants, les déodorants, les astringents, les produits rafraîchissants, les toniques les cicatrisants, les kératolytiques, les dépilatoires, les eaux parfumées, les extraits de tissus végétaux, les colorants hydrosolubles, les agents anti-pelliculaires, les agents anti-séborrhéiques, les oxydants, les réducteurs, les vitamines, les hormones, les enzymes, les vaccins, les anti-inflammatoires, les antibiotiques, les bactéricides et les agents cytotoxiques ou antitumoraux.

**15.** Composition sous forme de dispersion aqueuse de sphérules lipidiques selon l'une quelconque des revendications 10 à 13, caractérisée par le fait qu'elle contient au moins une substance active liposoluble choisie parmi les filtres solaires liposolubles, les substances destinées à améliorer l'état des peaux sèches ou séniles, les tocophérols, les vitamines E, F ou A et leurs esters, l'acide rétinoïque, les antioxydants, les acides gras essentiels, l'acide glycyrrhétinique, les kératolytiques et les caroténoïdes.

**16.** Composition sous forme de dispersion aqueuse de sphérules lipidiques selon l'une quelconque des revendications 10 à 15, caractérisée par le fait qu'elle comprend 2 à 70% en poids d'une phase liquide non miscible à l'eau choisie parmi les huiles, les hydrocarbures, les hydrocarbures halogénés, la perfluorotributylamine, les polysiloxanes, les esters d'acides organiques, les éthers et polyéthers.

**17.** Utilisation du céramide selon l'une quelconque des revendications 1 à 4, en tant que constituant cireux ayant des propriétés émollientes et adoucissantes dans des émulsions, des dispersions, des gels, des bâtonnets solides, des sprays, des mousses aérosol ou dans des lotions à usage cosmétique ou dermopharmaceutique.

**18.** Utilisation du céramide selon l'une quelconque des revendications 1 à 4 en association avec au moins un autre composé lipidique, pour la formation de dispersions de sphérules lipidiques à usage cosmétique ou dermopharmaceutique.

**19.** Procédé de traitement cosmétique de la peau, des cheveux ou des poils, caractérisé par le fait qu'il consiste à appliquer sur la peau, les cheveux ou les poils une quantité suffisante d'une composition selon l'une quelconque des revendications 7 à 16.

**Revendications pour l'Etat contractant suivant : ES**

**1.** Procédé de préparation d'un céramide comprenant un composé de formule (I) suivante :

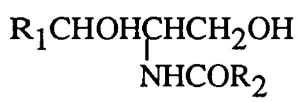 (I)

dans laquelle :

. $R_1$ désigne un radical alcoyle ou alcényle en $C_{11}$ à $C_{21}$;

. $R_2$ désigne un radical hydrocarboné en $C_{11}$-$C_{19}$, linéaire et portant une ou plusieurs insaturations éthyléniques, et notamment une ou deux, ou un mélange de composés de formule (I) et de formule

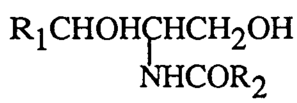 (Ia)

dans laquelle $R_1$ est défini comme précédemment et $R_2$ est un radical hydrocarboné linéaire saturé en $C_{11}$-$C_{19}$, la proportion de composés de formule (Ia) dans le mélange n'excédant pas 35%,

ce céramide étant sous la forme d'un mélange racémique des diastéréoisomères érythro et thréo dans des proportions érythro:thréo de 85:15 à 60:40,

caractérisé par le fait qu'il consiste à acyler la fonction amine d'une sphingosine, ou d'une dihydrosphingosine de formule (II) suivante

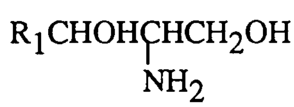 (II)

dans laquelle $R_1$ désigne un radical alcoyle ou alcényle en $C_{11}$ à $C_{21}$, ce composé étant sous forme de mélange racémique des diastéréoisomères érythro et thréo dans les proportions érythro:thréo de 85:15 à 60:40,

par un chlorure d'acide, par un anhydride, par un ester de paranitrophénol, par un ester de succinimide, par un ester de dicyclohexylcarbodiimide, par un ester d'alkyle inférieur ou par un azolide, en milieu anhydre ou dans des solvants tels que le tétrahydrofuranne, la pyridine, le diméthylformamide et le dichlorométhane.

**2.** Procédé de préparation d'un composé de formule (I) selon la revendication 1, caractérisé par le fait que l'anhydride est un anhydride mixte de formule $R_2COOCOOC_2H_5$, $R_2$ ayant la signification indiquée dans la revendication 1, l'ester d'alkyle inférieur est un ester de méthyle ou d'éthyle et l'azolide est un

imidazolide ou un pyrazolide.

3. Procédé selon la revendication 1, caractérisé par le fait que $R_1$ est un radical en $C_{15}$ dérivé de sphingosine, ou dihydrosphingosine.

4. Procédé selon la revendication 1 ou 2, caractérisé par le fait que $R_2$ est un radical dérivé de l'acide oléique ou linoléique.

5. Procédé selon l'une quelconque des revendications 1 à 4, caractérisé par le fait qu'on prépare un composé de formule (I) choisi parmi le 2-oléoylamino-1,3-octadécanediol et le 2-linoléoylamino-1,3-octadécanediol.

6. Composition à usage cosmétique ou dermopharmaceutique, caractérisée par le fait qu'elle contient 0,05 à 20% en poids, de préférence 0,1 à 10% en poids, d'au moins un céramide comprenant un composé de formule (I) suivante :

$$\begin{array}{l} R_1CHOH\underset{|}{C}HCH_2OH \\ \qquad\quad\;\; NHCOR_2 \end{array} \qquad (I)$$

dans laquelle :
- . $R_1$ désigne un radical alcoyle ou alcényle en $C_{11}$ à $C_{21}$;
- . $R_2$ désigne un radical hydrocarboné en $C_{11}$-$C_{19}$, linéaire et portant une ou plusieurs insaturations éthyléniques, et notamment une ou deux, ou un mélange de composés de formule (I) et de formule

$$\begin{array}{l} R_1CHOH\underset{|}{C}HCH_2OH \\ \qquad\quad\;\; NHCOR_2 \end{array} \qquad (Ia)$$

dans laquelle $R_1$ est défini comme précédemment et $R_2$ est un radical hydrocarboné linéaire saturé en $C_{11}$-$C_{19}$, la proportion de composés de formule (Ia) dans le mélange n'excédant pas 35%,

ce céramide étant sous la forme d'un mélange racémique des diastéréoisomères érythro et thréo dans des proportions érythro:thréo de 85:15 à 60:40, en présence d'un adjuvant choisi parmi les corps gras, les solvants, l'eau, les épaississants, les émulsionnants, les produits hydratants, les adoucissants, les filtres solaires, les germicides, les colorants, les conservateurs, les parfums, les propulseurs et les tensio-actifs.

7. Composition selon la revendication 6, caractérisée par le fait qu'elle se présente sous forme d'émulsion dont la phase grasse, représentant 5 à 60% du poids total de l'émulsion, est essentiellement constituée d'un mélange du ou des céramides avec au moins une huile, la phase aqueuse constituant 30 à 85% du poids total de l'émulsion, l'agent émulsionnant étant présent à raison de 1 à 20% en poids, et de préférence 2 à 12% en poids par rapport au poids total de l'émulsion.

8. Composition selon la revendication 6, caractérisée par le fait qu'elle se présente sous forme de lotion huileuse, oléoalcoolique ou hydroalcoolique, sous forme de gel, de dispersion, de bâtonnets solides, de spray ou de mousse aérosol.

9. Composition selon la revendication 6, caractérisée par le fait qu'elle se présente sous forme d'une dispersion aqueuse de sphérules lipidiques, constituées de couches moléculaires organisées renfermant une phase aqueuse encapsulée, ces couches étant constituées du ou des céramides associés à au moins un autre composé lipidique.

10. Composition sous forme de dispersion aqueuse de sphérules lipidiques selon la revendication 9, caractérisée par le fait que l'autre composé lipidique est choisi parmi les alcools et diols à longue

chaîne, les stérols, les phospholipides, les glycolipides, les cholestéryl sulfate et phosphate, les amines à longue chaîne et leurs dérivés d'ammonium quaternaire, les dihydroxyalkylamines, les amines grasses polyoxyéthylénées, les esters d'aminoalcools à longue chaîne, leurs sels et dérivés d'ammonium quaternaire, les esters phosphoriques d'alcools gras, les alkylsulfates et les acides gras sous forme de sels.

**11.** Composition sous forme de dispersion aqueuse de sphérules lipidiques selon la revendication 9 ou 10, caractérisée par le fait que les sphérules ont un diamètre compris entre 0,05µm et 5µm.

**12.** Composition sous forme de dispersion aqueuse de sphérules lipidiques selon la revendication 9 ou 10, caractérisée par le fait que la phase aqueuse encapsulée dans les sphérules est de l'eau ou une solution aqueuse de substance active.

**13.** Composition sous forme de dispersion aqueuse de sphérules lipidiques selon l'une quelconque des revendications 9 à 12, caractérisée par le fait que la phase aqueuse encapsulée dans les sphérules contient au moins une substance active hydrosoluble cosmétique et/ou pharmaceutique choisie parmi les humectants, les agents de brunissage artificiel éventuellement associés à des colorants, les filtres solaires hydrosolubles, les antiperspirants, les déodorants, les astringents, les produits rafraîchissants, les toniques, les cicatrisants, les kératolytiques, les dépilatoires, les eaux parfumées, les extraits de tissus végétaux, les colorants hydrosolubles, les agents anti-pelliculaires, les agents anti-séborrhéiques, les oxydants, les réducteurs, les vitamines, les hormones, les enzymes, les vaccins, les anti-inflammatoires, les antibiotiques, les bactéricides et les agents cytotoxiques ou antitumoraux.

**14.** Composition sous forme de dispersion aqueuse de sphérules lipidiques selon l'une quelconque des revendications 9 à 12, caractérisée par le fait qu'elle contient au moins une substance active liposoluble choisie parmi les filtres solaires liposolubles, les substances destinées à améliorer l'état des peaux sèches ou séniles, les tocophérols, les vitamines E, F ou A et leurs esters, l'acide rétinoïque, les antioxydants, les acides gras essentiels, l'acide glycyrrhétinique, les kératolytiques et les caroténoïdes.

**15.** Composition sous forme de dispersion aqueuse de sphérules lipidiques selon l'une quelconque des revendications 9 à 14, caractérisée par le fait qu'elle comprend 2 à 70% en poids d'une phase liquide non miscible à l'eau choisie parmi les huiles, les hydrocarbures, les hydrocarbures halogénés, la perfluorotributylamine, les polysiloxanes, les esters d'acides organiques, les éthers et polyéthers.

**16.** Procédé de préparation d'une composition à usage cosmétique ou dermopharmaceutique caractérisé par le fait qu'on introduit 0,05 à 20% en poids, de préférence 0,1 à 10% en poids, d'au moins un céramide comprenant un composé de formule (I) suivante :

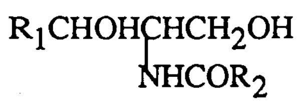

(I)

dans laquelle :

. $R_1$ désigne un radical alcoyle ou alcényle en $C_{11}$ à $C_{21}$;
. $R_2$ désigne un radical hydrocarboné en $C_{11}$-$C_{19}$, linéaire et portant une ou plusieurs insaturations éthyléniques, et notamment une ou deux, ou un mélange de composés de formule (I) et de formule

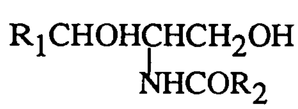

(Ia)

dans laquelle $R_1$ est défini comme précédemment et $R_2$ est un radical hydrocarboné linéaire saturé en $C_{11}$-$C_{19}$, la proportion de composés de formule (Ia) dans le mélange n'excédant pas 35%,

ce céramide étant sous la forme d'un mélange racémique des diastéréoisomères érythro et

thréo dans des proportions érythro:thréo de 85:15 à 60:40 dans un support comprenant des adjuvants choisis parmi les corps gras, les solvants, l'eau, les épaississants, les émulsionnants, les produits hydratants, les adoucissants, les filtres solaires, les germicides, les colorants, les conservateurs, les parfums, les propulseurs et les tensio-actifs.

**17.** Procédé selon la revendication 16 de préparation d'une composition à usage cosmétique ou dermopharmaceutique sous forme d'une dispersion aqueuse de sphérules lipidiques constituées de couches moléculaires organisées renfermant une phase aqueuse encapsulée, caractérisé par le fait qu'on utilise pour la formation de ces couches, le ou les céramides associés à au moins un autre composé lipidique.

**18.** Procédé de préparation d'une dispersion aqueuse de sphérules lipidiques selon la revendication 17, caractérisé par le fait qu'on introduit dans la phase aqueuse encapsulée dans les sphérules au moins une substance hydrosoluble ayant une activité cosmétique et/ou pharmaceutique choisie parmi les humectants, les agents de brunissage artificiel éventuellement associés à des colorants, les filtres solaires hydrosolubles, les antiperspirants, les déodorants, les astringents, les produits rafraîchissants, les toniques, les cicatrisants, les kératolytiques, les dépilatoires, les eaux parfumées, les extraits de tissus végétaux, les colorants hydrosolubles, les agents antipelliculaires, les agents anti-séborrhéiques, les oxydants, les réducteurs, les vitamines, les hormones, les enzymes, les vaccins, les anti-inflammatoires, les antibiotiques, les bactéricides et les agents cytotoxiques ou antitumoraux.

**19.** Procédé de préparation d'une dispersion aqueuse de sphérules lipidiques selon la revendication 17, caractérisé par le fait qu'on introduit dans les couches moléculaires organisées des sphérules lipidiques, au moins une substance active liposoluble choisie parmi les filtres solaires liposolubles, les substances destinées à améliorer l'état des peaux sèches ou séniles, les tocophérols, les vitamines E, F ou A et leurs esters, l'acide rétinoïque, les antioxydants, les acides gras essentiels, l'acide glycyrrhétinique, les kératolytiques et les caroténoïdes.

**Revendications pour l'Etat contractant suivant : GR**

**1.** Céramide synthétique comprenant un composé de formule :

$$\begin{array}{l} R_1CHOHCHCH_2OH \\ \qquad\quad\;\; | \\ \qquad\quad NHCOR_2 \end{array} \qquad (I)$$

dans laquelle :

. $R_1$ désigne un radical alcoyle ou alcényle en $C_{11}$ à $C_{21}$;

. $R_2$ désigne un radical hydrocarboné en $C_{11}$-$C_{19}$, linéaire et portant une ou plusieurs insaturations éthyléniques, et notamment une ou deux, ou un mélange de composés de formule (I) et de formule :

$$\begin{array}{l} R_1CHOHCHCH_2OH \\ \qquad\quad\;\; | \\ \qquad\quad NHCOR_2 \end{array} \qquad (Ia)$$

dans laquelle $R_1$ est défini comme précédemment et $R_2$ est un radical hydrocarboné linéaire saturé en $C_{11}$-$C_{19}$, la proportion de composés de formule (Ia) dans le mélange n'excédant pas 35%,

ce céramide étant sous la forme d'un mélange racémique des diastéréoisomères érythro et thréo dans des proportions érythro:thréo de 85:15 à 60:40.

**2.** Céramide synthétique selon la revendication 1, caractérisé par le fait que $R_1$ est un radical en $C_{15}$ dérivé de sphingosine, ou dihydrosphingosine.

**3.** Céramide synthétique selon la revendication 1 ou 2, caractérisé par le fait que $R_2$ est un radical dérivé de l'acide oléique ou linoléique.

**4.** Céramide synthétique selon l'une quelconque des revendications 1 à 3, caractérisé par le fait qu'il est choisi parmi le 2-oléoylamino-1,3-octadécanediol ou le 2-linoléoylamino-1,3-octadécanediol.

**5.** Procédé de préparation d'un céramide selon l'une quelconque des revendications 1 à 4, caractérisé par le fait qu'il consiste à acyler la fonction amine d'une sphingosine, ou d'une dihydrosphingosine de formule (II) suivante

$$\underset{\phantom{CHOH}NH_2}{R_1CHOHCHCH_2OH} \qquad (II)$$

dans laquelle $R_1$ désigne un radical alcoyle ou alcényle en $C_{11}$ à $C_{21}$, ce composé étant sous forme de mélange racémique des diastéréoisomères érythro et thréo dans les proportions érythro:thréo de 85:15 à 60:40,
par un chlorure d'acide, par un anhydride, par un ester de paranitrophénol, par un ester de succinimide, par un ester de dicyclohexylcarbodiimide, par un ester d'alkyle inférieur ou par un azolide, en milieu anhydre ou dans des solvants tels que le tétrahydrofuranne, la pyridine, le diméthylformamide et le dichlorométhane.

**6.** Procédé de préparation d'un céramide selon la revendication 1, caractérisé par le fait que l'anhydride est un anhydride mixte de formule $R_2COOCOOC_2H_5$, $R_2$ ayant la signification
indiquée dans la revendication 1, l'ester d'alkyle inférieur est un ester de méthyle ou d'éthyle et l'azolide est un imidazolide ou un pyrazolide.

**7.** Composition à usage cosmétique ou dermopharmaceutique, caractérisée par le fait qu'elle contient 0,05 à 20% en poids, de préférence 0,1 à 10% en poids, d'au moins un céramide selon l'une quelconque des revendications 1 à 4, en présence d'un adjuvant choisi parmi les corps gras, les solvants, l'eau, les épaississants, les émulsionnants, les produits hydratants, les adoucissants, les filtres solaires, les germicides, les colorants, les conservateurs, les parfums, les propulseurs et les tensioactifs.

**8.** Composition selon la revendication 7, caractérisée par le fait qu'elle se présente sous forme d'émulsion dont la phase grasse, représentant 5 à 60% du pois total de l'émulsion, est essentiellement constituée d'un mélange du céramide avec au moins une huile, la phase aqueuse constituant 30 à 85% du poids total de l'émulsion, l'agent émulsionnant étant présent à raison de 1 à 20% en poids, et de préférence 2 à 12% en poids par rapport au poids total de l'émulsion.

**9.** Composition selon la revendication 7, caractérisée par le fait qu'elle se présente sous forme de lotion huileuse, oléoalcoolique ou hydroalcoolique, sous forme de gel, de dispersion, de bâtonnets solides, de spray ou de mousse aérosol.

**10.** Composition selon la revendication 7, caractérisée par le fait qu'elle se présente sous forme d'une dispersion aqueuse de sphérules lipidiques, constituées de couches moléculaires organisées renfermant une phase aqueuse encapsulée, ces couches étant constituées du ou des céramides associés à au moins un autre composé lipidique.

**11.** Composition sous forme de dispersion aqueuse de sphérules lipidiques selon la revendication 10, caractérisée par le fait que l'autre composé lipidique est choisi parmi les alcools et diols à longue chaîne, les stérols, les phospholipides, les glycolipides, les cholestéryl sulfate et phosphate, les amines à longue chaîne et leurs dérivés d'ammonium quaternaire, les dihydroxyalkylamines, les amines grasses polyoxyéthylénées, les esters d'aminoalcools à longue chaîne, leurs sels et dérivés d'ammonium quaternaire, les esters phosphoriques d'alcools gras, les alkylsulfates et les acides gras sous forme de sels.

**12.** Composition sous forme de dispersion aqueuse de sphérules lipidiques selon la revendication 10 ou 11, caractérisée par le fait que les sphérules ont un diamètre compris entre 0,05µm et 5µm.

**13.** Composition sous forme de dispersion aqueuse de sphérules lipidiques selon la revendication 10 ou 11, caractérisée par le fait que la phase aqueuse encapsulée dans les sphérules est de l'eau ou une solution aqueuse de substance active.

**14.** Composition sous forme de dispersion aqueuse de sphérules lipidiques selon l'une quelconque des revendications 10 à 13, caractérisée par le fait que la phase aqueuse encapsulée dans les sphérules contient au moins une substance active hydrosoluble cosmétique et/ou pharmaceutique choisie parmi les humectants, les agents de brunissage artificiel éventuellement associés à des colorants, les filtres solaires hydrosolubles, les antiperspirants, les déodorants, les astringents, les produits rafraîchissants, les toniques les cicatrisants, les kératolytiques, les dépilatoires, les eaux parfumées, les extraits de tissus végétaux, les colorants hydrosolubles, les agents anti-pelliculaires, les agents anti-séborrhéiques, les oxydants, les réducteurs, les vitamines, les hormones, les enzymes, les vaccins, les anti-inflammatoires, les antibiotiques, les bactéricides et les agents cytotoxiques ou antitumoraux.

**15.** Composition sous forme de dispersion aqueuse de sphérules lipidiques selon l'une quelconque des revendications 10 à 13, caractérisée par le fait qu'elle contient au moins une substance active liposoluble choisie parmi les filtres solaires liposolubles, les substances destinées à améliorer l'état des peaux sèches ou séniles, les tocophérols, les vitamines E, F ou A et leurs esters, l'acide rétinoïque, les antioxydants, les acides gras essentiels, l'acide glycyrrhétinique, les kératolytiques et les caroténoïdes.

**16.** Composition sous forme de dispersion aqueuse de sphérules lipidiques selon l'une quelconque des revendications 10 à 15, caractérisée par le fait qu'elle comprend 2 à 70% en poids d'une phase liquide non miscible à l'eau choisie parmi les huiles, les hydrocarbures, les hydrocarbures halogénés, la perfluorotributylamine, les polysiloxanes, les esters d'acides organiques, les éthers et polyéthers.

**17.** Procédé de préparation d'une composition à usage cosmétique ou dermopharmaceutique caractérisé par le fait qu'on introduit 0,05 à 20% en poids, de préférence 0,1 à 10% en poids, d'au moins un céramide comprenant un composé de formule (I) suivante :

$$\begin{array}{l} R_1CHOHCHCH_2OH \\ \qquad\qquad\quad | \\ \qquad\qquad\; NHCOR_2 \end{array} \qquad (I)$$

dans laquelle :

. $R_1$ désigne un radical alcoyle ou alcényle en $C_{11}$ à $C_{21}$;

. $R_2$ désigne un radical hydrocarboné en $C_{11}$-$C_{19}$, linéaire et portant une ou plusieurs insaturations éthyléniques, et notamment une ou deux, ou un mélange de composés de formule (I) et de formule :

$$\begin{array}{l} R_1CHOHCHCH_2OH \\ \qquad\qquad\quad | \\ \qquad\qquad\; NHCOR_2 \end{array} \qquad (Ia)$$

dans laquelle $R_1$ est défini comme précédemment et $R_2$ est un radical hydrocarboné linéaire saturé en $C_{11}$-$C_{19}$, la proportion de composés de formule (Ia) dans le mélange n'excédant pas 35%,

ce céramide étant sous la forme d'un mélange racémique des diastéréoisomères érythro et thréo dans des proportions érythro:thréo de 85:15 à 60:40, dans un support comprenant des adjuvants choisis parmi les corps gras, les solvants, l'eau, les épaississants, les émulsionnants, les produits hydratants, les adoucissants, les filtres solaires, les germicides, les colorants, les conservateurs, les parfums, les propulseurs et les tensio-actifs.

**18.** Procédé selon la revendication 17 de préparation d'une composition à usage cosmétique ou dermopharmaceutique sous forme d'une dispersion aqueuse de sphérules lipidiques constituées de couches moléculaires organisées renfermant une phase aqueuse encapsulée, caractérisé par le fait qu'on utilise pour la formation de ces couches, un composé de formule (I) associé à au moins un autre composé lipidique.

**19.** Procédé de préparation d'une dispersion aqueuse de sphérules lipidiques selon la revendication 18, caractérisé par le fait qu'on introduit dans la phase aqueuse encapsulée dans les sphérules au moins une substance hydrosoluble à activité cosmétique et/ou pharmaceutique choisie parmi les humectants, les agents de brunissage artificiel éventuellement associés à des colorants, les filtres solaires hydrosolubles, les antiperspirants, les déodorants, les astringents, les produits rafraîchissants, les toniques les cicatrisants, les kératolytiques, les dépilatoires, les eaux parfumées, les extraits de tissus végétaux, les colorants hydrosolubles, les agents anti-pelliculaires, les agents anti-séborrhéiques, les oxydants, les réducteurs, les vitamines, les hormones, les enzymes, les vaccins, les anti-inflammatoires, les antibiotiques, les bactéricides et les agents cytotoxiques ou antitumoraux.

**20.** Procédé de préparation d'une dispersion aqueuse de sphérules lipidiques selon la revendication 18, caractérisé par le fait qu'on introduit dans les couches moléculaires organisées des sphérules lipidiques, au moins une substance active liposoluble choisie parmi les filtres solaires hydrosolubles, les substances destinées à améliorer l'état des peaux sèches ou séniles, les tocophérols, les vitamines E, F ou A et leurs esters, l'acide rétinoïque, les antioxydants, les acides gras essentiels, l'acide glycyrrhétinique, les kératolytiques et les caroténoïdes.

**Claims**

**Claims for the following Contracting States : AT, BE, CH, DE, DK, FR, GB, IT, LI, NL, PT, SE**

**1.** Synthetic ceramide comprising a compound of formula:

$$\begin{array}{l} R_1CHOH\underset{\displaystyle NHCOR_2}{\underset{|}{C}}HCH_2OH \end{array} \qquad (I)$$

in which:

- . $R_1$ denotes a $C_{11}$ to $C_{21}$ alkyl or alkenyl radical;
- . $R_2$ denotes a $C_{11}$-$C_{19}$ hydrocarbon radical, which is linear and carries one or more ethylenic unsaturations, and in particular one or two, or a mixture of compounds of formula (I) and of formula:

$$\begin{array}{l} R_1CHOH\underset{\displaystyle NHCOR_2}{\underset{|}{C}}HCH_2OH \end{array} \qquad (Ia)$$

in which $R_1$ is defined as above and $R_2$ is a saturated linear $C_{11}$-$C_{19}$ hydrocarbon radical, the proportion of compounds of formula (Ia) in the mixture not exceeding 35%,

this ceramide being in the form of a racemic mixture of the *erythro* and *threo* diastereoisomers in the *erythro:threo* proportions of 85:15 to 60:40.

**2.** Synthetic ceramide according to Claim 1, characterised in that $R_1$ is a $C_{15}$ radical derived from sphingosine or dihydrosphingosine.

**3.** Synthetic ceramide according to either of Claims 1 and 2, characterised in that $R_2$ is a radical derived from oleic or linoleic acid.

**4.** Synthetic ceramide according to any one of Claims 1 to 3, characterised in that it is chosen from 2-oleoylamino-1,3-octadecanediol and 2-linoleoylamino-1,3-octadecanediol.

**5.** Process for preparing a ceramide according to any one of Claims 1 to 4, characterised in that it consists in acylating an amine functional group of a sphingosine or a dihydrosphingosine of the following formula (II)

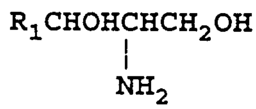

(II)

in which $R_1$ denotes a $C_{11}$ to $C_{21}$ alkyl or alkenyl radical, this compound being in the form of a racemic mixture of the *erythro* and *threo* diastereoisomers in the *erythro: threo* proportions of 85:15 to 60:40, with an acid chloride, with an anhydride, with a paranitrophenol ester, with a succinimide ester, with a dicyclohexylcarbodiimide ester, with a lower alkyl ester or with an azolide, in an anhydrous medium or in solvents such as tetrahydrofuran, pyridine, dimethylformamide and dichloromethane.

**6.** Process for preparing a ceramide according to Claim 5; characterised in that the anhydride is a mixed anhydride of formula $R_2COOCOOC_2H_5$, $R_2$ having the meaning given in Claim 1, the lower alkyl ester is a methyl or ethyl ester and the azolide is an imidazolide or a pyrazolide.

**7.** Composition for cosmetic or dermopharmaceutical use, characterised in that it contains 0.05 to 20% by weight, preferably 0.1 to 10% by weight, of at least one ceramide according to any one of Claims 1 to 4, in the presence of an adjuvant chosen from fatty substances, solvents, water, thickeners, emulsifiers, moisturising products, demulcents, sunscreen agents, germicides, colorants, preservatives, perfumes, propellants and surface-active agents.

**8.** Composition according to Claim 7, characterised in that it is provided in the form of an emulsion whose fatty phase, representing 5 to 60% of the total weight of the emulsion, is essentially composed of a mixture of the ceramide with at least one oil, the aqueous phase constituting 30 to 85% of the total weight of the emulsion, the emulsifying agent being present in an amount of 1 to 20% by weight, and preferably 2 to 12% by weight relative to the total weight of the emulsion.

**9.** Composition according to Claim 7, characterised in that it is provided in the form of an oily, oil-alcohol or dilute alcoholic lotion, in the form of a gel, a dispersion, solid sticks, a spray or an aerosol foam.

**10.** Composition according to Claim 7, characterised in that it is provided in the form of an aqueous dispersion of lipid spherules composed of organised molecular layers enclosing an encapsulated aqueous phase, these layers being composed of the ceramide or ceramides combined with at least one other lipid compound.

**11.** Composition in the form of an aqueous dispersion of lipid spherules according to Claim 10, characterised in that the other lipid compound is chosen from long chain alcohols and diols, sterols, phospholipids, glycolipids, cholesteryl sulphate and phosphate, long chain amines and quaternary ammonium derivatives thereof, dihydroxyalkylamines, polyoxyethylenated fatty amines, long chain amino alcohol esters, quaternary ammonium salts and derivatives thereof, fatty alcohol phosphoric esters, alkyl sulphates and fatty acids in the form of salts.

**12.** Composition in the form of an aqueous dispersion of lipid spherules according to either of Claims 10 and 11, characterised in that the spherules have a diameter of between 0.05 $\mu$m and 5 $\mu$m.

**13.** Composition in the form of an aqueous dispersion of lipid spherules according to either of Claims 10 and 11, characterised in that the aqueous phase encapsulated in the spherules is water or an aqueous solution of active substance.

**14.** Composition in the form of an aqueous dispersion of lipid spherules according to any one of Claims 10 to 13, characterised in that the aqueous phase encapsulated in the spherules contains at least one

cosmetic and/or pharmaceutical, water-soluble active substance chosen from humectants, artificial tanning agents optionally combined with colorants, water-soluble sunscreen agents, antiperspiration agents, deodorants, astringents, refreshing products, tonic products, cicatrising products, keratolytic agents, depilatory products, perfumed water, plant tissue extracts, water-soluble colorants, antidandruff agents, antiseborrhoeic agents, oxidants, reducing agents, vitamins, hormones, enzymes, vaccines, anti-inflammatory agents, antibiotics, bactericides and cytotoxic or antitumour agents.

**15.** Composition in the form of an aqueous dispersion of lipid spherules according to any one of Claims 10 to 13, characterised in that it contains at least one fat-soluble active substance chosen from fat-soluble sunscreen agents, substances intended to improve the condition of dry or aged skin, tocopherols, vitamins E, F or A and esters thereof, retinoic acid, antioxidants, essential fatty acids, glycyrrhetinic acid, keratolytic agents and carotenoides.

**16.** Composition in the form of an aqueous dispersion of lipid spherules according to any one of Claims 10 to 15, characterised in that it comprises 2 to 70% by weight of a water-immiscible liquid phase chosen from oils, hydrocarbons, halogenated hydrocarbons, perfluorotributylamine, polysiloxanes, organic acid esters, ethers and polyethers.

**17.** Use of the ceramide according to any one of Claims 1 to 4, as a waxy constituent having emollient and demulcent properties in emulsions, dispersions, gels, solid sticks, sprays, aerosol foams or in lotions for cosmetic or dermopharmaceutical use.

**18.** Use of the ceramide according to any one of Claims 1 to 4 in combination with at least one other lipid compound, for the formation of dispersions of lipid spherules for cosmetic or dermopharmaceutical use.

**19.** Process for the cosmetic treatment of the skin, the hair or hair strands, characterised in that it consists in applying to the skin, the hair or the hair strands a sufficient amount of a composition according to any one of Claims 7 to 16.

**Claims for the following Contracting State : ES**

**1.** Process for preparing a ceramide comprising a compound of the following formula (I):

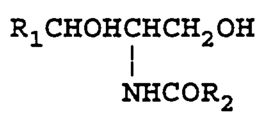

(I)

in which:

- . $R_1$ denotes a $C_{11}$ to $C_{21}$ alkyl or alkenyl radical;
- . $R_2$ denotes a $C_{11}$-$C_{19}$ hydrocarbon radical, which is linear and carries one or more ethylenic unsaturations, and in particular one or two, or a mixture of compounds of formula (I) and of formula

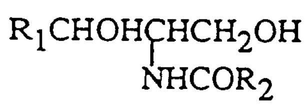

(Ia)

in which $R_1$ is defined as above and $R_2$ is a saturated, linear $C_{11}$-$C_{19}$ hydrocarbon radical, the proportion of compounds of formula (Ia) in the mixture not exceeding 35%,

this ceramide being in the form of a racemic mixture of the *erythro* and *threo* diastereoisomers in the *erythro:threo* proportions of 85:15 to 60:40, characterised in that it consists in acylating an amine functional group of a sphingosine or a dihydrosphingosine of the following formula (II)

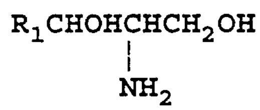

(II)

in which $R_1$ denotes a $C_{11}$ to $C_{21}$ alkyl or alkenyl radical, this compound being in the form of a racemic mixture of the *erythro* and *threo* diastereoisomers in the *erythro: threo* proportions of 85:15 to 60:40,

with an acid chloride, with an anhydride, with a paranitrophenol ester, with a succinimide ester, with a dicyclohexylcarbodiimide ester, with a lower alkyl ester or with an azolide, in an anhydrous medium or in solvents such as tetrahydrofuran, pyridine, dimethylformamide and dichloromethane.

**2.** Process for preparing a compound of formula (I) according to Claim 1, characterised in that the anhydride is a mixed anhydride of formula $R_2COOCOOC_2H_5$, $R_2$ having the meaning given in Claim 1, the lower alkyl ester is a methyl or ethyl ester and the azolide is an imidazolide or a pyrazolide.

**3.** Process according to Claim 1, characterised in that $R_1$ is a $C_{15}$ radical derived from sphingosine or dihydrosphingosine.

**4.** Process according to either of Claims 1 and 2, characterised in that $R_2$ is a radical derived from oleic or linoleic acid.

**5.** Process according to any one of Claims 1 to 4, characterised in that a compound of formula (I) is prepared which is chosen from 2-oleoylamino-1,3-octadecanediol and 2-linoleoylamino-1,3-octadecanediol.

**6.** Composition for cosmetic or dermopharmaceutical use, characterised in that it contains 0.05 to 20% by weight, preferably 0.1 to 10% by weight, of at least one ceramide comprising a compound of the following formula (I):

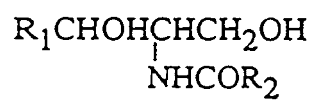

(I)

in which:

$R_1$ denotes a $C_{11}$ to $C_{21}$ alkyl or alkenyl radical;

$R_2$ denotes a $C_{11}$-$C_{19}$ hydrocarbon radical, which is linear and carries one or more ethylenic unsaturations, and in particular one or two, or a mixture of compounds of formula (I) and of formula

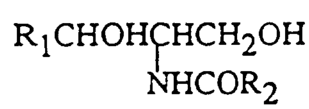

(Ia)

in which $R_1$ is defined as above and $R_2$ is a saturated linear $C_{11}$-$C_{19}$ hydrocarbon radical, the proportion of compounds of formula (Ia) in the mixture not exceeding 35%,

this ceramide being in the form of a racemic mixture of the *erythro* and *threo* diastereoisomers in the *erythro:threo* proportions of 85:15 to 60:40, in the presence of an adjuvant chosen from fatty substances, solvents, water, thickeners, emulsifiers, moisturising products, demulcents, sunscreen agents, germicides, colorants, preservatives, perfumes, propellants and surface-active agents.

**7.** Composition according to Claim 6, characterised in that it is provided in the form of an emulsion whose fatty phase, representing 5 to 60% of the total weight of the emulsion, is essentially composed of a mixture of the ceramide or ceramides with at least one oil, the aqueous phase constituting 30 to 85% of the total weight of the emulsion, the emulsifying agent being present in an amount of 1 to 20% by

weight, and preferably 2 to 12% by weight relative to the total weight of the emulsion.

**8.** Composition according to Claim 6, characterised in that it is provided in the form of an oily, oil-alcohol or dilute alcoholic lotion, in the form of a gel, a dispersion, solid sticks, a spray or an aerosol foam.

**9.** Composition according to Claim 6, characterised in that it is provided in the form of an aqueous dispersion of lipid spherules composed of organised molecular layers enclosing an encapsulated aqueous phase, these layers being composed of the ceramide or ceramides combined with at least one other lipid compound.

**10.** Composition in the form of an aqueous dispersion of lipid spherules according to Claim 9, characterised in that the other lipid compound is chosen from long chain alcohols and diols, sterols, phospholipids, glycolipids, cholesteryl sulphate and phosphate, long chain amines and quaternary ammonium derivatives thereof, dihydroxyalkylamines, polyoxyethylenated fatty amines, long chain amino alcohol esters, quaternary ammonium salts and derivatives thereof, fatty alcohol phosphoric esters, alkyl sulphates and fatty acids in the form of salts.

**11.** Composition in the form of an aqueous dispersion of lipid spherules according to either of Claims 9 and 10, characterised in that the spherules have a diameter of between 0.05 µm and 5 µm.

**12.** Composition in the form of an aqueous dispersion of lipid spherules according to either of Claims 9 and 10, characterised in that the aqueous phase encapsulated in the spherules is water or an aqueous solution of active substance.

**13.** Composition in the form of an aqueous dispersion of lipid spherules according to any one of Claims 9 to 12, characterised in that the aqueous phase encapsulated in the spherules contains at least one cosmetic and/or pharmaceutical, water-soluble active substance chosen from humectants, artificial tanning agents optionally combined with colorants, water-soluble sunscreen agents, antiperspiration agents, deodorants, astringents, refreshing products, tonic products, cicatrising products, keratolytic agents, depilatory products, perfumed water, plant tissue extracts, water-soluble colorants, antidandruff agents, antiseborrhoeic agents, oxidants, reducing agents, vitamins, hormones, enzymes, vaccines, anti-inflammatory agents, antibiotics, bactericides and cytotoxic or antitumour agents.

**14.** Composition in the form of an aqueous dispersion of lipid spherules according to any one of Claims 9 to 12, characterised in that it contains at least one fat-soluble active substance chosen from fat-soluble sunscreen agents, substances intended to improve the condition of dry or aged skin, tocopherols, vitamins E, F or A and esters thereof, retinoic acid, antioxidants, essential fatty acids, glycyrrhetinic acid, keratolytic agents and carotenoides.

**15.** Composition in the form of an aqueous dispersion of lipid spherules according to any one of Claims 9 to 14, characterised in that it comprises 2 to 70% by weight of a water-immiscible liquid phase chosen from oils, hydrocarbons, halogenated hydrocarbons, perfluorotributylamine, polysiloxanes, organic acid esters, ethers and polyethers.

**16.** Process for preparing a composition for cosmetic or dermopharmaceutical use, characterised in that there is introduced 0.05 to 20% by weight, preferably 0.1 to 10% by weight, of at least one ceramide comprising a compound of the following formula (I):

$$\begin{array}{l} R_1CHOHCHCH_2OH \\ \qquad\qquad\quad | \\ \qquad\qquad\quad NHCOR_2 \end{array} \qquad (I)$$

in which:

$R_1$ denotes a $C_{11}$ to $C_{21}$ alkyl or alkenyl radical;

$R_2$ denotes a $C_{11}$-$C_{19}$ hydrocarbon radical, which is linear and carries one or more ethylenic unsaturations, and in particular one or two, or a mixture of compounds of formula (I) and of formula

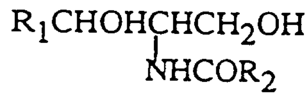

(Ia)

in which $R_1$ is defined as above and $R_2$ is a saturated linear $C_{11}$-$C_{19}$ hydrocarbon radical, the proportion of compounds of formula (Ia) in the mixture not exceeding 35%, this ceramide being in the form of a racemic mixture of the *erythro* and *threo* diastereoisomers in *erythro:threo* proportions of 85:15 to 60:40, in a carrier comprising adjuvants chosen from fatty substances, solvents, water, thickeners, emulsifiers, moisturising products, demulcents, sunscreen agents, germicides, colorants, preservatives, perfumes, propellants and surface-active agents.

**17.** Process according to Claim 16, for preparing a composition for cosmetic or dermopharmaceutical use in the form of an aqueous dispersion of lipid spherules composed of organised molecular layers enclosing an encapsulated aqueous phase, characterised in that the ceramide or ceramides combined with at least one other lipid compound is (are) used to form these layers.

**18.** Process for preparing an aqueous dispersion of lipid spherules according to Claim 17, characterised in that there is introduced into the aqueous phase encapsulated in the spherules at least one water-soluble substance having a cosmetic and/or pharmaceutical activity chosen from humectants, artificial tanning agents optionally combined with colorants, water-soluble sunscreen agents, antiperspiration agents, deodorants, astringents, refreshing products, tonic products, cicatrising products, keratolytic agents, depilatory products, perfumed waters, plant tissue extracts, water-soluble colorants, antidandruff agents, antiseborrhoeic agents, oxidants, reducing agents, vitamins, hormones, enzymes, vaccines, anti-inflammatory agents, antibiotics, bactericides and cytotoxic or antitumour agents.

**19.** Process for preparing an aqueous dispersion of lipid spherules according to Claim 17, characterised in that there is introduced into the organised molecular layers of lipid spherules at least one fat-soluble active substance chosen from fat-soluble sunscreen agents, substances intended to improve the condition of dry or aged skin, tocopherols, vitamins E, F or A and esters thereof, retinoic acid, antioxidants, essential fatty acids, glycyrrhetinic acid, keratolytic agents and carotenoids.

**Claims for the following Contracting State : GR**

**1.** Synthetic ceramide comprising a compound of formula:

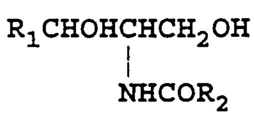

(I)

in which:

. $R_1$ denotes a $C_{11}$ to $C_{21}$ alkyl or alkenyl radical;

. $R_2$ denotes a $C_{11}$-$C_{19}$ hydrocarbon radical, which is linear and carries one or more ethylenic unsaturations, and in particular one or two, or a mixture of compounds of formula (I) and of formula:

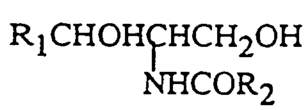

(Ia)

in which $R_1$ is defined as above and $R_2$ is a saturated linear $C_{11}$-$C_{19}$ hydrocarbon radical, the proportion of compounds of formula (Ia) in the mixture not exceeding 35%,

this ceramide being in the form of a racemic mixture of the *erythro* and *threo* diastereoisomers in the *erythro:threo* proportions of 85:15 to 60:40.

**2.** Synthetic ceramide according to Claim 1, characterised in that $R_1$ is a $C_{15}$ radical derived from sphingosine or dihydrosphingosine.

**3.** Synthetic ceramide according to either of Claims 1 and 2, characterised in that $R_2$ is a radical derived from oleic or linoleic acid.

**4.** Synthetic ceramide according to any one of Claims 1 to 3, characterised in that it is chosen from 2-oleoylamino-1,3-octadecanediol and 2-linoleoylamino-1,3-octadecanediol.

**5.** Process for preparing a ceramide according to any one of Claims 1 to 4, characterised in that it consists in acylating an amine functional group of a sphingosine or a dihydrosphingosine of the following formula (II)

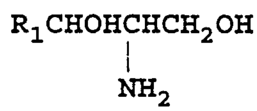

(II)

in which $R_1$ denotes a $C_{11}$ to $C_{21}$ alkyl or alkenyl radical, this compound being in the form of a racemic mixture of the *erythro* and *threo* diastereoisomers in the *erythro:threo* proportions of 85:15 to 60:40, with an acid chloride, with an anhydride, with a paranitrophenol ester, with a succinimide ester, with a dicyclohexylcarbodiimide ester, with a lower alkyl ester or with an azolide, in an anhydrous medium or in solvents such as tetrahydrofuran, pyridine, dimethylformamide and dichloromethane.

**6.** Process for preparing a ceramide according to Claim 1, characterised in that the anhydride is a mixed anhydride of formula $R_2COOCOOC_2H_5$, $R_2$ having the meaning given in Claim 1, the lower alkyl ester is a methyl or ethyl ester and the azolide is an imidazolide or a pyrazolide.

**7.** Composition for cosmetic or dermopharmaceutical use, characterised in that it contains 0.05 to 20% by weight, preferably 0.1 to 10% by weight, of at least one ceramide according to any one of Claims 1 to 4, in the presence of an adjuvant chosen from fatty substances, solvents, water, thickeners, emulsifiers, moisturising products, demulcents, sunscreen agents, germicides, colorants, preservatives, perfumes, propellants and surface-active agents.

**8.** Composition according to Claim 7, characterised in that it is provided in the form of an emulsion whose fatty phase, representing 5 to 60% of the total weight of the emulsion, is essentially composed of a mixture of the ceramide with at least one oil, the aqueous phase constituting 30 to 85% of the total weight of the emulsion, the emulsifying agent being present in an amount of 1 to 20% by weight, and preferably 2 to 12% by weight relative to the total weight of the emulsion.

**9.** Composition according to Claim 7, characterised in that it is provided in the form of an oily, oil-alcohol or dilute alcoholic lotion, in the form of a gel, a dispersion, solid sticks, a spray or an aerosol foam.

**10.** Composition according to Claim 7, characterised in that it is provided in the form of an aqueous dispersion of lipid spherules composed of organised molecular layers enclosing an encapsulated aqueous phase, these layers being composed of the ceramide or ceramides combined with at least one other lipid compound.

**11.** Composition in the form of an aqueous dispersion of lipid spherules according to Claim 10, characterised in that the other lipid compound is chosen from long chain alcohols and diols, sterols, phospholipids, glycolipids, cholesteryl sulphate and phosphate, long chain amines and quaternary ammonium derivatives thereof, dihydroxyalkylamines, polyoxyethylenated fatty amines, long chain amino alcohol esters, quaternary ammonium salts and derivatives thereof, fatty alcohol phosphoric esters, alkyl sulphates and fatty acids in the form of salts.

**12.** Composition in the form of an aqueous dispersion of lipid spherules according to either of Claims 10 and 11, characterised in that the spherules have a diameter of between 0.05 $\mu m$ and 5 $\mu m$.

**13.** Composition in the form of an aqueous dispersion of lipid spherules according to either of Claims 10 and 11, characterised in that the aqueous phase encapsulated in the spherules is water or an aqueous solution of active substance.

**14.** Composition in the form of an aqueous dispersion of lipid spherules according to any one of Claims 10 to 13, characterised in that the aqueous phase encapsulated in the spherules contains at least one cosmetic and/or pharmaceutical, water-soluble active substance chosen from humectants, artificial tanning agents optionally combined with colorants, water-soluble sunscreen agents, antiperspiration agents, deodorants, astringents, refreshing products, tonic products, cicatrising products, keratolytic agents, depilatory products, perfumed water, plant tissue extracts, water-soluble colorants, antidandruff agents, antiseborrhoeic agents, oxidants, reducing agents, vitamins, hormones, enzymes, vaccines, anti-inflammatory agents, antibiotics, bactericides and cytotoxic or antitumour agents.

**15.** Composition in the form of an aqueous dispersion of lipid spherules according to any one of Claims 10 to 13, characterised in that it contains at least one fat-soluble active substance chosen from fat-soluble sunscreen agents, substances intended to improve the condition of dry or aged skin, tocopherols, vitamins E, F or A and esters thereof, retinoic acid, antioxidants, essential fatty acids, glycyrrhetinic acid, keratolytic agents and carotenoides.

**16.** Composition in the form of an aqueous dispersion of lipid spherules according to any one of Claims 10 to 15, characterised in that it comprises 2 to 70% by weight of a water-immiscible liquid phase chosen from oils, hydrocarbons, halogenated hydrocarbons, perfluorotributylamine, polysiloxanes, organic acid esters, ethers and polyethers.

**17.** Process for preparing a composition for cosmetic or dermopharmaceutical use, characterised in that there is introduced 0.05 to 20% by weight, preferably 0.1 to 10% by weight, of at least one ceramide comprising a compound of the following formula (I):

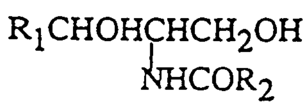

(I)

in which:

$R_1$ denotes a $C_{11}$ to $C_{21}$ alkyl or alkenyl radical;

$R_2$ denotes a $C_{11}$-$C_{19}$ hydrocarbon radical, which is linear and carries one or more ethylenic unsaturations, and in particular one or two, or a mixture of compounds of formula (I) and of formula

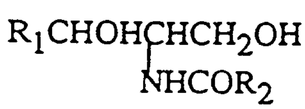

(Ia)

in which $R_1$ is defined as above and $R_2$ is a saturated linear $C_{11}$-$C_{19}$ hydrocarbon radical, the proportion of compounds of formula (Ia) in the mixture not exceeding 35%,

this ceramide being in the form of a racemic mixture of the *erythro* and *threo* diastereoisomers in *erythro:threo* proportions of 85:15 to 60:40, in a carrier comprising adjuvants chosen from fatty substances, solvents, water, thickeners, emulsifiers, moisturising products, demulcents, sunscreen agents, germicides, colorants, preservatives, perfumes, propellants and surface-active agents.

**18.** Process according to Claim 17, for preparing a composition for cosmetic or dermopharmaceutical use in the form of an aqueous dispersion of lipid spherules composed of organised molecular layers enclosing an encapsulated aqueous phase, characterised in that a compound of formula (I) combined

with at least one other lipid compound is (are) used to form these layers.

**19.** Process for preparing an aqueous dispersion of lipid spherules according to Claim 18, characterised in that there is introduced into the aqueous phase encapsulated in the spherules at least one water-soluble substance having a cosmetic and/or pharmaceutical activity chosen from humectants, artificial tanning agents optionally combined with colorants, water-soluble sunscreen agents, antiperspiration agents, deodorants, astringents, refreshing products, tonic products, cicatrising products, keratolytic agents, depilatory products, perfumed waters, plant tissue extracts, water-soluble colorants, antidandruff agents, antiseborrhoeic agents, oxidants, reducing agents, vitamins, hormones, enzymes, vaccines, anti-inflammatory agents, antibiotics, bactericides and cytotoxic or antitumour agents.

**20.** Process for preparing an aqueous dispersion of lipid spherules according to Claim 18, characterised in that there is introduced into the organised molecular layers of lipid spherules at least one fat-soluble active substance chosen from water-soluble sunscreen agents, substances intended to improve the condition of dry or aged skin, tocopherols, vitamins E, F or A and esters thereof, retinoic acid, antioxidants, essential fatty acids, glycyrrhetinic acid, keratolytic agents and carotenoids.

**Patentansprüche**
**Patentansprüche für folgende Vertragsstaaten : AT, BE, CH, DE, DK, FR, GB, IT, LI, NL, PT, SE**

**1.** Synthetisches Ceramid, enthaltend eine Verbindung der Formel:

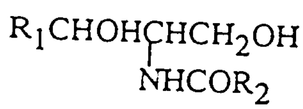
(I)

worin gilt:
- $R_1$ bedeutet einen $C_{11-21}$-Alkyl- oder -Alkenylrest,
- $R_2$ bedeutet einen linearen $C_{11-19}$-Kohlenwasserstoffrest, der eine oder mehrere, insbesondere eine oder zwei, ethylenisch ungesättigte Bindungen aufweist,

oder enthaltend eine Mischung von Verbindungen der Formel (I) sowie der Formel:

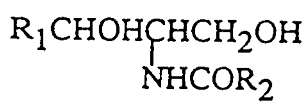
(Ia)

worin $R_1$ wie vorher definiert und $R_2$ ein linearer gesättigter $C_{11-19}$-Kohlenwasserstoffrest sind, wobei der Mengenanteil der Verbindungen der Formel (Ia) in der Mischung 35% nicht übersteigt, wobei das Ceramid in Form einer racemischen Mischung der erythro- und threo-Diastereoisomeren in erythro:threo-Mengenverhältnissen von 85:15 bis 60:40 vorliegt.

**2.** Synthetisches Ceramid gemäß Beispiel 1,
dadurch **gekennzeichnet**, daß
$R_1$ ein $C_{15}$-Rest ist, der von Sphingosin oder Dihydrosphingosin abgeleitet ist.

**3.** Synthetisches Ceramid gemäß Anspruch 1 oder 2,
dadurch **gekennzeichnet**, daß
$R_2$ ein Rest ist, der von Öl- oder Linolensäure abgeleitet ist.

**4.** Synthetisches Ceramid gemäß einem jeden der Ansprüche 1 bis 3,
dadurch **gekennzeichnet**, daß
es aus 2-Oleoylaminooctadecan-1,3-diol und aus 2-Linoleoylaminooctadecan-1,3-diol ausgewählt ist.

**5.** Verfahren zur Herstellung eines Ceramids gemäß einem jeden der Ansprüche 1 bis 4,
dadurch **gekennzeichnet**, daß

man die Aminfunktion eines Sphingosins oder Dihydroshingosins der folgenden Formel (II):

$$R_1CHOHCHCH_2OH$$
$$NH_2 \qquad (II)$$

worin $R_1$ einen $C_{11-21}$-Alkyl- oder -Alkenylrest darstellt, wobei diese Verbindung in Form einer racemischen Mischung der erythro- und threo-Diastereoisomeren in erythro:threo-Mengenanteilen von 85:15 bis 60:40 vorliegt,
mit einem Säurechlorid, Säureanhydrid, p-Nitrophenolester, Succinimidester, Dicyclohexylcarbodiimidester,
Niedrigalkylester oder mit einem Azolid in wasserfreiem Milieu oder in Lösungsmitteln wie Tetrahydrofuran, Pyridin, Dimethylformamid und Dichlormethan acyliert.

**6.** Verfahren zur Herstellung eines Ceramids gemäß Anspruch 5,
dadurch **gekennzeichnet**, daß
das Säureanhydrid ein gemischtes Anhydrid der Formel $R_2COOCOOC_2H_5$, worin $R_2$ die in Anspruch 1 angegebene Bedeutung hat, der Niedrigalkylester ein Methyl- oder Ethylester und das Azolid ein Imidazolid oder Pyrazolid sind.

**7.** Zusammensetzung zur kosmetischen oder dermopharmazeutischen Verwendung,
dadurch **gekennzeichnet**, daß
sie 0,05 bis 20, vorzugsweise 0,1 bis 10, Gew.% mindestens eines Ceramids gemäß einem jeden der Ansprüche 1 bis 4 in Gegenwart eines Hilfsstoffes enthält, der aus Fettkörpern, Lösungsmitteln, Wasser, Verdickungsmitteln, Emulgatoren, hydratisierenden Produkten, weichmachenden Mitteln, Sonnenfilterstoffen, Germiciden, Färbemitteln, Konservierungsstoffen, Parfüm-Produkten, Treibmitteln und oberflächenaktiven Mitteln ausgewählt ist.

**8.** Zusammensetzung gemäß Anspruch 7,
dadurch **gekennzeichnet**, daß
sie in Form einer Emulsion vorliegt, deren Fettphase, die 5 bis 60% des Gesamtgewichts der Emulsion ausmacht, im wesentlichen aus einer Mischung des Ceramids mit mindestens einem Öl zusammengesetzt ist, wobei die wässrige Phase 30 bis 85% des Gesamtgewichts der Emulsion ausmacht, und wobei ein Emulgator mit 1 bis 20 und vorzugsweise mit 2 bis 12 Gew.% vorhanden ist, bezogen auf das Gesamtgewicht der Emulsion.

**9.** Zusammensetzung gemäß Anspruch 7,
dadurch **gekennzeichnet**, daß
sie in Form einer öligen, oleoalkoholischen oder hydroalkoholischen Lotion, in Form eines Gels, einer Dispersion, von Feststoffstäbchen, eines Spray-Produkts oder eines Aerosol-Schaums vorliegt.

**10.** Zusammensetzung gemäß Anspruch 7,
dadurch **gekennzeichnet**, daß
sie in Form einer wässrigen Dispersion lipidischer, Kügelchen aus geordneten Molekularschichten, die eine eingekapselte wässrige Phase umschließen, vorliegt, wobei diese Schichten aus dem oder den Ceramid(en) zusammengesetzt sind, welche zusammen mit mindestens einer weiteren lipidischen Verbindung vorliegen.

**11.** Zusammensetzung in Form einer wässrigen Dispersion von lipidischen Kügelchen gemäß Anspruch 10,
dadurch **gekennzeichnet**, daß
die weitere lipidische Verbindung aus Alkoholen und Diolen mit langer Kette, Sterolen, Phospholipiden, Glycolipiden, Cholesterylsulfat und -phosphat, Aminen mit langer Kette und deren quaternären Ammoniumderivaten, Dihydroxylaminen, polyoxethylierten Fettaminen, Aminoalkoholestern mit langer Kette, deren Salzen und quaternären Ammoniumderivaten, Phosphorsäureestern von Fettalkoholen, Alkylsulfaten sowie aus Fettsäuren in Form von Salzen ausgewählt ist.

12. Zusammensetzung in Form einer wässrigen Dispersion lipidischer Kügelchen gemäß Anspruch 10 oder 11,
dadurch **gekennzeichnet**, daß
die Kügelchen einen Durchmesser von 0,05 bis 5 $\mu$m aufweisen.

13. Zusammensetzung in Form einer wässrigen Dispersion lipidischer Kügelchen gemäß Anspruch 10 oder 11,
dadurch **gekennzeichnet**, daß
die in den Kügelchen eingekapselte wässrige Phase Wasser oder eine wässrige Lösung einer Wirksubstanz ist.

14. Zusammensetzung in Form einer wässrigen Dispersion lipidischer Kügelchen gemäß einem jeden der Ansprüche 10 bis 13,
dadurch **gekennzeichnet**, daß
die in den Kügelchen eingekapselte wässrige Phase mindestens eine wasserlösliche kosmetische und/oder pharmazeutische Wirksubstanz enthält, ausgewählt aus Feuchtigkeitsmitteln, künstlichen Bräunungsmitteln, die gegebenenfalls zusammen mit Färbemitteln vorliegen, aus wasserlöslichen Sonnenfilterstoffen, Antiausdünnmitteln, Deodoriermitteln, zusammenziehenden Mitteln, Erfrischungsprodukten, tonischen Mitteln, Linderungsmitteln, Keratolytika, Enthaarungsmitteln, parfümierten Wässern, Extrakten von pflanzlichen Geweben, wasserlöslichen Färbemitteln, Mitteln gegen Haarausfall, antiseborrheischen Mitteln, oxidierenden Mitteln, reduzierenden Mitteln, Vitaminen, Hormonen, Enzymen, Impfstoffen, entzündungshemmenden Mitteln, Antibiotika, Bakteriziden sowie aus cytotoxischen und antitumoralen Mitteln.

15. Zusammensetzung in Form einer wässrigen Dispersion lipidischer Kügelchen gemäß einem jeden der Ansprüche 10 bis 13,
dadurch **gekennzeichnet**, daß
sie mindestens eine fettlösliche Aktivsubstanz enthält, ausgewählt aus fettlöslichen Sonnenfilterstoffen, Substanzen zur Verbesserung des Zustands trockener oder gealterter Hautbereiche, aus Tocopherolen, Vitaminen E, F oder A und deren Estern, aus Retinoesäure, Antioxidantien, essentiellen Fettsäuren, Glycyrrhetinsäure, Keratolytika und Carotinoiden.

16. Zusammensetzung in Form einer wässrigen Dispersion lipidischer Kügelchen gemäß einem jeden der Ansprüche 10 bis 15,
dadurch **gekennzeichnet**, daß
sie 2 bis 70 Gew.% einer mit Wasser nicht mischbaren flüssigen Phase enthält, die aus Ölen, Kohlenwasserstoffen, halogenierten Kohlenwasserstoffen, Perfluortributylamin, Polysiloxanen, Estern organischer Säuren, Ethern und Polyethern ausgewählt ist.

17. Verwendung des Ceramids gemäß einem jeden der Ansprüche 1 bis 4 als wachsartiger Bestandteil, welcher erweichende und glättende Eigenschaften in Emulsionen, Dispersionen, Gelen, Feststoffstäbchen, Spray-Produkten, Aerosol-Schäumen oder in Lotionen zum kosmetischen oder dermopharmazeutischen Gebrauch aufweist.

18. Verwendung des Ceramids gemäß einem jeden der Ansprüche 1 bis 4 zusammen mit mindestens einer weiteren lipidischen Verbindung zur Bildung von Dispersionen lipidischer Kügelchen zum kosmetischen oder dermopharmazeutischen Gebrauch.

19. Verfahren zur kosmetischen Behandlung der Haut, der Haare oder von Behaarungen,
dadurch **gekennzeichnet**, daß
man auf die Haut, die Haare oder die Behaarungen eine ausreichende Menge einer Zusammensetzung gemäß einem jeden der Ansprüche 7 bis 16 aufträgt.

**Patentansprüche für folgenden Vertragsstaat : ES**

1. Verfahren zur Herstellung eines Ceramid, enthaltend eine Verbindung der Formel (I):

$$R_1CHOHCHCH_2OH \quad (I)$$
$$NHCOR_2$$

worin gilt:
- $R_1$ bedeutet einen $C_{11-21}$-Alkyl- oder -Alkenylrest,
- $R_2$ bedeutet einen linearen $C_{11-19}$-Kohlenwasserstoffrest, der eine oder mehrere, insbesondere eine oder zwei, ethylenisch ungesättigte Bindungen aufweist,
oder enthaltend eine Mischung von Verbindungen der Formel (I) sowie der Formel:

$$R_1CHOHCHCH_2OH \quad (Ia)$$
$$NHCOR_2$$

worin $R_1$ wie vorher definiert und $R_2$ ein linearer gesättigter $C_{11-19}$-Kohlenwasserstoffrest sind, wobei der Mengenanteil der Verbindungen der Formel (Ia) in der Mischung 35% nicht übersteigt, wobei das Ceramid in Form einer racemischen Mischung der erythro- und threo-Diastereoisomeren in erythro:threo-Mengenverhältnissen von 85:15 bis 60:40 vorliegt,
dadurch **gekennzeichnet**, daß
man die Amninfunktion eines Sphingosins oder Dihydroshingosins der folgenden Formel (II):

$$R_1CHOHCHCH_2OH$$
$$NH_2 \quad (II)$$

worin $R_1$ einen $C_{11-21}$-Alkyl- oder -Alkenylrest darstellt, wobei diese Verbindung in Form einer racemischen Mischung der erythro- und threo-Diastereoisomeren in erythro:threo-Mengenanteilen von 85:15 bis 60:40 vorliegt,
mit einem Säurechlorid, Säureanhydrid, p-Nitrophenolester, Succinimidester, Dicyclohexylcarbodiimidester, Niedrigalkylester oder mit einem Azolid in wasserfreiem Milieu oder in Lösungsmitteln wie Tetrahydrofuran, Pyridin, Dimethylformamid und Dichlormethan acyliert.

2. Verfahren zur Herstellung einer Verbindung der Formel (I) gemäß Anspruch 1,
dadurch **gekennzeichnet**, daß
das Säureanhydrid ein gemischtes Anhydrid der Formel $R_2COOCOOC_2H_5$, worin $R_2$ die in Anspruch 1 angegebene Bedeutung hat, der Niedrigalkylester ein Methyl- oder Ethylester und das Azolid ein Imidazolid oder Pyrazolid sind.

3. Verfahren gemäß Anspruch 1,
dadurch **gekennzeichnet**, daß
$R_1$ ein $C_{15}$-Rest ist, der von Sphingosin oder Dihydrosphingosin abgeleitet ist.

4. Verfahren gemäß Anspruch 1 oder 2,
dadurch **gekennzeichnet**, daß
$R_2$ ein Rest ist, der von Öl- oder Linolensäure abgeleitet ist.

5. Verfahren gemäß einem jeden der Ansprüche 1 bis 4,
dadurch **gekennzeichnet**, daß
man eine Verbindung der Formel (I) herstellt, die aus 2-Oleoylaminooctadecan-1,3-diol und aus 2-Linoleoylaminooctadecan-1,3-diol ausgewählt ist.

**6.** Zusammensetzung zur kosmetischen oder dermopharmazeutischen Verwendung,
dadurch **gekennzeichnet**, daß
sie 0,05 bis 20 und vorzugsweise 0,1 bis 10 Gew.% eines Ceramids, enthaltend eine Verbindung der Formel (I):

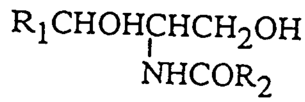

(I)

worin gilt:

- $R_1$ bedeutet einen $C_{11-21}$-Alkyl- oder -Alkenylrest,
- $R_2$ bedeutet einen linearen $C_{11-19}$-Kohlenwasserstoffrest, der eine oder mehrere, insbesondere eine oder zwei, ethylenisch ungesättigte Bindungen aufweist,
  oder enthaltend eine Mischung von Verbindungen der Formel (I) sowie der Formel:

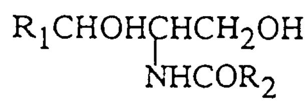

(Ia)

worin $R_1$ wie vorher definiert und $R_2$ ein linearer gesättigter $C_{11-19}$-Kohlenwasserstoffrest sind, wobei der Mengenanteil der Verbindungen der Formel (Ia) in der Mischung 35% nicht übersteigt, wobei das Ceramid in Form einer racemischen Mischung der erythro- und threo-Diastereoisomeren in erythro:threo-Mengenverhältnissen von 85:15 bis 60:40 vorliegt, in Gegenwart eines Hilfsstoffes aufweist, ausgewählt aus Fettkorpern, Lösungsmitteln, Wasser, Verdickungsmitteln, Emulgatoren, hydratisierenden Produkten, weichmachenden Mitteln, Sonnenfilterstoffen, Germiziden, Färbemitteln, Konservierungsstoffen, Parfüm-Produkten, Treibmitteln und aus oberflächenaktiven Mitteln.

**7.** Zusammensetzung gemäß Anspruch 6,
dadurch **gekennzeichnet**, daß
sie in Form einer Emulsion vorliegt, deren Fettphase, die 5 bis 60% des Gesamtgewichts der Emulsion ausmacht, im wesentlichen aus einer Mischung des Ceramids mit mindestens einem Öl zusammengesetzt ist, wobei die wässrige Phase 30 bis 85% des Gesamtgewichts der Emulsion ausmacht, und wobei ein Emulgator mit 1 bis 20 und vorzugsweise mit 2 bis 12 Gew.% vorhanden ist, bezogen auf das Gesamtgewicht der Emulsion.

**8.** Zusammensetzung gemäß Anspruch 6,
dadurch **gekennzeichnet**, daß
sie in Form einer öligen, oleoalkoholischen oder hydroalkoholischen Lotion, in Form eines Gels, einer Dispersion, von Feststoffstäbchen, eines Spray-Produkts oder eines Aerosol-Schaums vorliegt.

**9.** Zusammensetzung gemäß Anspruch 6,
dadurch **gekennzeichnet**, daß
sie in Form einer wässrigen Dispersion lipidischer Kügelchen aus geordneten Molekularschichten, die eine eingekapselte wässrige Phase umschließen, vorliegt, wobei diese Schichten aus dem oder den Ceramid(en) zusammengesetzt sind, welche zusammen mit mindestens einer weiteren lipidischen Verbindung vorliegen.

**10.** Zusammensetzung in Form einer wässrigen Dispersion von lipidischen Kügelchen gemäß Anspruch 9,
dadurch **gekennzeichnet**, daß
die weitere lipidische Verbindung aus Alkoholen und Diolen mit langer Kette, Sterolen, Phospholipiden, Glycolipiden, Cholesterylsulfat und -phosphat, Aminen mit langer Kette und deren quaternären Ammoniumderivaten, Dihydroxylaminen, polyoxethylierten Fettaminen, Aminoalkoholestern mit langer Kette, deren Salzen und quaternären Ammoniumderivaten, Phosphorsäureestern von Fettalkoholen, Alkylsulfaten sowie aus Fettsäuren in Form von Salzen ausgewählt ist.

**11.** Zusammensetzung in Form einer wässrigen Dispersion lipidischer Kügelchen gemäß Anspruch 9 oder 10, dadurch **gekennzeichnet**, daß die Kügelchen einen Durchmesser von 0,05 bis 5 µm aufweisen.

**12.** Zusammensetzung in Form einer wässrigen Dispersion lipidischer Kügelchen gemäß Anspruch 9 oder 10, dadurch **gekennzeichnet**, daß die in den Kügelchen eingekapselte wässrige Phase Wasser oder eine wässrige Lösung einer Wirksubstanz ist.

**13.** Zusammensetzung in Form einer wässrigen Dispersion lipidischer Kügelchen gemäß einem jeden der Ansprüche 10 bis 13, dadurch **gekennzeichnet**, daß die in den Kügelchen eingekapselte wässrige Phase mindestens eine wasserlösliche kosmetische und/oder pharmazeutische Wirksubstanz enthält, ausgewählt aus Feuchtigkeitsmitteln, künstlichen Bräunungsmitteln, die gegebenenfalls zusammen mit Färbemitteln vorliegen, aus wasserlöslichen Sonnenfilterstoffen, Antiausdünnmitteln, Deodoriermitteln, zusammenziehenden Mitteln, Erfrischungsprodukten, tonischen Mitteln, Linderungsmitteln, Keratolytika, Enthaarungsmitteln, parfümierten Wässern, Extrakten von pflanzlichen Geweben, wasserlöslichen Färbemitteln, Mitteln gegen Haarausfall, antiseborrheischen Mitteln, oxidierenden Mitteln, reduzierenden Mitteln, Vitaminen, Hormonen, Enzymen, Impfstoffen, entzündungshemmenden Mitteln, Antibiotika, Bakteriziden sowie aus cytotoxischen und antitumoralen Mitteln.

**14.** Zusammensetzung in Form einer wässrigen Dispersion lipidischer Kügelchen gemäß einem jeden der Ansprüche 9 bis 12, dadurch **gekennzeichnet**, daß sie mindestens eine fettlösliche Aktivsubstanz enthält, ausgewählt aus fettlöslichen Sonnenfilterstoffen, Substanzen zur Verbesserung des Zustands trockener oder gealterter Hautbereiche, aus Tocopherolen, Vitaminen E, F oder A und deren Estern, aus Retinoesäure, Antioxidantien, essentiellen Fettsäuren, Glycyrrhetinsäure, Keratolytika und Carotinoiden.

**15.** Zusammensetzung in Form einer wässrigen Dispersion lipidischer Kügelchen gemäß einem jeden der Ansprüche 9 bis 14, dadurch **gekennzeichnet**, daß sie 2 bis 70 Gew.% einer mit Wasser nicht mischbaren flüssigen Phase enthält, die aus Ölen, Kohlenwasserstoffen, halogenierten Kohlenwasserstoffen, Perfluortributylamin, Polysiloxanen, Estern organischer Säuren, Ethern und Polyethern ausgewählt ist.

**16.** Verfahren zur Herstellung einer Zusammensetzung zur kosmetischen oder dermopharmazeutischen Verwendung, dadurch **gekennzeichnet**, daß man 0,05 bis 20 und vorzugsweise 0,1 bis 10 Gew.% mindestens eines Ceramids, enthaltend eine Verbindung der Formel (I):

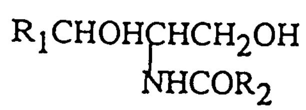

(I)

worin gilt:

- $R_1$ bedeutet einen $C_{11-21}$-Alkyl- oder -Alkenylrest,
- $R_2$ bedeutet einen linearen $C_{11-19}$-Kohlenwasserstoffrest, der eine oder mehrere, insbesondere eine oder zwei, ethylenisch ungesättigte Bindungen aufweist,

  oder enthaltend eine Mischung von Verbindungen der Formel (I) sowie der Formel:

$$\begin{array}{l} R_1CHOHCHCH_2OH \\ \qquad\quad\;\; | \\ \qquad\quad\;\; NHCOR_2 \end{array} \qquad (Ia)$$

worin $R_1$ wie vorher definiert und $R_2$ ein linearer gesättigter $C_{11-19}$-Kohlenwasserstoffrest sind, wobei der Mengenanteil der Verbindungen der Formel (Ia) in der Mischung 35% nicht übersteigt, wobei das Ceramid in Form einer racemischen Mischung der erythro- und threo-Diastereoisomeren in erythro:threo-Mengenverhältnissen von 85:15 bis 60:40 vorliegt, in ein Trägermedium einbringt, das Hilsstoffe enthält, ausgewählt aus Fettkörpern, Lösungsmitteln, Wasser, Verdikkungsmitteln, Emulgatoren, hydratisierenden Produkten, weichmachenden Mitteln, Sonnenfilterstoffen, Germiziden, Färbemitteln, Konservierungsstoffen, Parfüm-Produkten, Treibmitteln und aus oberflächenaktiven Mitteln.

**17.** Verfahren gemäß Anspruch 16 zur Herstellung einer Zusammensetzung zur kosmetischen oder dermopharmazeutichen Verwendung in Form einer wässrigen Dispersion lipidischer Kügelchen aus geordneten Molekularschichten, die eine eingekapselte wässrige Phase umschließen,
dadurch **gekennzeichnet**, daß
man zur Bildung dieser Schichten das oder die Ceramide zusammen mit mindestens einer weiteren lipidischen Verbindung verwendet.

**18.** Verfahren zur Herstellung einer wässrigen Dispersion lipidischer Kügelchen gemäß Anspruch 17,
dadurch **gekennzeichnet**, daß
man in die in die Kügelchen eingekapselte wässrige Phase mindestens eine wasserlösliche Substanz einbringt, die eine kosmetische und/oder pharmazeutische Wirksamkeit aufweist und aus Feuchtigkeitsmitteln, künstlichen Bräunungsmitteln, die gegebenenfalls zusammen mit Färbemitteln vorliegen, aus wasserlöslichen Sonnenfilterstoffen, Antiausdünnmitteln, Deodoriermitteln, zusammenziehenden Mitteln, Erfrischungsprodukten, tonischen Mitteln, Linderungsmitteln, Keratolytika, Enthaarungsmitteln, parfümierten Wässern, Extrakten von pflanzlichen Geweben, wasserlöslichen Färbemitteln, Mitteln gegen Haarausfall, antiseborrheischen Mitteln, oxidierenden Mitteln, reduzierenden Mitteln, Vitaminen, Hormonen, Enzymen, Impfstoffen, entzündungshemmenden Mitteln, Antibiotika, Bakteriziden sowie aus cytotoxischen und antitumoralen Mitteln ausgewählt ist.

**19.** Verfahren zur Herstellung einer wässrigen Dispersion lipidischer Kügelchen gemäß Anspruch 17,
dadurch **gekennzeichnet**, daß
man in die geordneten Molekularschichten der lipidischen Kügelchen mindestens eine fettlösliche Aktivsubstanz einbringt, ausgewählt aus fettlöslichen Sonnenfilterstoffen, Substanzen zur Verbesserung des Zustands trockener oder gealterter Hautbereiche, aus Tocopherolen, Vitaminen E, F oder A und deren Estern, aus Retinoesäure, Antioxidantien, essentiellen Fettsäuren, Glycyrrhetinsäure, Keratolytika und Carotinoiden.

**Patentansprüche für folgenden Vertragsstaat : GR**

**1.** Synthetisches Ceramid, enthaltend eine Verbindung der Formel:

$$\begin{array}{l} R_1CHOHCHCH_2OH \\ \qquad\quad\;\; | \\ \qquad\quad\;\; NHCOR_2 \end{array} \qquad (I)$$

worin gilt:
- $R_1$ bedeutet einen $C_{11-21}$-Alkyl- oder -Alkenylrest,
- $R_2$ bedeutet einen linearen $C_{11-19}$-Kohlenwasserstoffrest, der eine oder mehrere, insbesondere eine oder zwei, ethylenisch ungesättigte Bindungen aufweist,
oder enthaltend eine Mischung von Verbindungen der Formel (I) sowie der Formel:

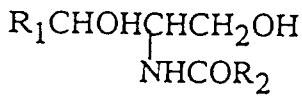

(Ia)

worin $R_1$ wie vorher definiert und $R_2$ ein linearer gesättigter $C_{11-19}$-Kohlenwasserstoffrest sind, wobei der Mengenanteil der Verbindungen der Formel (Ia) in der Mischung 35% nicht übersteigt, wobei das Ceramid in Form einer racemischen Mischung der erythro- und threo-Diastereoisomeren in erythro:threo-Mengenverhältnissen von 85:15 bis 60:40 vorliegt.

2. Synthetisches Ceramid gemäß Beispiel 1,
dadurch **gekennzeichnet**, daß
$R_1$ ein $C_{15}$-Rest ist, der von Sphingosin oder Dihydrosphingosin abgeleitet ist.

3. Synthetisches Ceramid gemäß Anspruch 1 oder 2,
dadurch **gekennzeichnet**, daß
$R_2$ ein Rest ist, der von Öl- oder Linolensäure abgeleitet ist.

4. Synthetisches Ceramid gemäß einem jeden der Ansprüche 1 bis 3,
dadurch **gekennzeichnet**, daß
es aus 2-Oleoylaminooctadecan-1,3-diol und aus 2-Linoleoylaminooctadecan-1,3-diol ausgewählt ist.

5. Verfahren zur Herstellung eines Ceramids gemäß einem jeden der Ansprüche 1 bis 4,
dadurch **gekennzeichnet**, daß
man die Aminfunktion eines Sphingosins oder Dihydroshingosins der folgenden Formel (II):

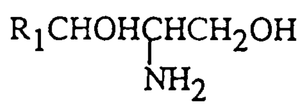

(II)

worin $R_1$ einen $C_{11-21}$-Alkyl- oder -Alkenylrest darstellt, wobei diese Verbindung in Form einer racemischen Mischung der erythro- und threo-Diastereoisomeren in erythro:threo-Mengenanteilen von 85:15 bis 60:40 vorliegt,
mit einem Säurechlorid, Säureanhydrid, p-Nitrophenolester, Succinimidester, Dicyclohexylcarbodiimidester, Niedrigalkylester oder mit einem Azolid in wasserfreiem Milieu oder in Lösungsmitteln wie Tetrahydrofuran, Pyridin, Dimethylformamid und Dichlormethan acyliert.

6. Verfahren zur Herstellung eines Ceramids gemäß Anspruch 5,
dadurch **gekennzeichnet**, daß
das Säureanhydrid ein gemischtes Anhydrid der Formel $R_2COOCOOC_2H_5$, worin $R_2$ die in Anspruch 1 angegebene Bedeutung hat, der Niedrigalkylester ein Methyl- oder Ethylester und das Azolid ein Imidazolid oder Pyrazolid sind.

7. Zusammensetzung zur kosmetischen oder dermopharmazeutischen Verwendung,
dadurch **gekennzeichnet**, daß
sie 0,05 bis 20, vorzugsweise 0,1 bis 10, Gew.% mindestens eines Ceramids gemäß einem jeden der Ansprüche 1 bis 4 in Gegenwart eines Hilfsstoffes enthält, der aus Fettkörpern, Lösungsmitteln, Wasser, Verdickungsmitteln, Emulgatoren, hydratisierenden Produkten, weichmachenden Mitteln, Sonnenfilterstoffen, Germiciden, Färbemitteln, Konservierungsstoffen, Parfüm-Produkten, Treibmitteln und oberflächenaktiven Mitteln ausgewählt ist.

8. Zusammensetzung gemäß Anspruch 7,
dadurch **gekennzeichnet**, daß
sie in Form einer Emulsion vorliegt, deren Fettphase, die 5 bis 60% des Gesamtgewichts der Emulsion ausmacht, im wesentlichen aus einer Mischung des Ceramids mit mindestens einem Öl zusammengesetzt ist, wobei die wässrige Phase 30 bis 85% des Gesamtgewichts der Emulsion ausmacht, und wobei ein Emulgator mit 1 bis 20 und vorzugsweise mit 2 bis 12 Gew.% vorhanden ist, bezogen auf das Gesamtgewicht der Emulsion.

**9.** Zusammensetzung gemäß Anspruch 7,
dadurch **gekennzeichnet**, daß
sie in Form einer öligen, oleoalkoholischen oder hydroalkoholischen Lotion, in Form eines Gels, einer Dispersion, von Feststoffstäbchen, eines Spray-Produkts oder eines Aerosol-Schaums vorliegt.

**10.** Zusammensetzung gemäß Anspruch 7,
dadurch **gekennzeichnet**, daß
sie in Form einer wässrigen Dispersion lipidischer Kügelchen aus geordneten Molekularschichten, die eine eingekapselte wässrige Phase umschließen, vorliegt, wobei diese Schichten aus dem oder den Ceramid(en) zusammengesetzt sind, welche zusammen mit mindestens einer weiteren lipidischen Verbindung vorliegen.

**11.** Zusammensetzung in Form einer wässrigen Dispersion von lipidischen Kügelchen gemäß Anspruch 10,
dadurch **gekennzeichnet**, daß
die weitere lipidische Verbindung aus Alkoholen und Diolen mit langer Kette, Sterolen, Phospholipiden, Glycolipiden, Cholesterylsulfat und -phosphat, Aminen mit langer Kette und deren quaternären Ammoniumderivaten, Dihydroxylaminen, polyoxethylierten Fettaminen, Aminoalkoholestern mit langer Kette, deren Salzen und quaternären Ammoniumderivaten, Phosphorsäureestern von Fettalkoholen, Alkylsulfaten sowie aus Fettsäuren in Form von Salzen ausgewählt ist.

**12.** Zusammensetzung in Form einer wässrigen Dispersion lipidischer Kügelchen gemäß Anspruch 10 oder 11,
dadurch **gekennzeichnet**, daß
die Kügelchen einen Durchmesser von 0,05 bis 5 $\mu$m aufweisen.

**13.** Zusammensetzung in Form einer wässrigen Dispersion lipidischer Kügelchen gemäß Anspruch 10 oder 11,
dadurch **gekennzeichnet**, daß
die in den Kügelchen eingekapselte wässrige Phase Wasser oder eine wässrige Lösung einer Wirksubstanz ist.

**14.** Zusammensetzung in Form einer wässrigen Dispersion lipidischer Kügelchen gemäß einem jeden der Ansprüche 10 bis 13,
dadurch **gekennzeichnet**, daß
die in den Kügelchen eingekapselte wässrige Phase mindestens eine wasserlösliche kosmetische und/oder pharmazeutische Wirksubstanz enthält, ausgewählt aus Feuchtigkeitsmitteln, künstlichen Bräunungsmitteln, die gegebenenfalls zusammen mit Färbemitteln vorliegen, aus wasserlöslichen Sonnenfilterstoffen, Antiausdünnmitteln, Deodoriermitteln, zusammenziehenden Mitteln, Erfrischungsprodukten, tonischen Mitteln, Linderungsmitteln, Keratolytika, Enthaarungsmitteln, parfümierten Wässern, Extrakten von pflanzlichen Geweben, wasserlöslichen Färbemitteln, Mitteln gegen Haarausfall, antiseborrheischen Mitteln, oxidierenden Mitteln, reduzierenden Mitteln, Vitaminen, Hormonen, Enzymen, Impfstoffen, entzündungshemmenden Mitteln, Antibiotika, Bakteriziden sowie aus cytotoxischen und antitumoralen Mitteln.

**15.** Zusammensetzung in Form einer wässrigen Dispersion lipidischer Kügelchen gemäß einem jeden der Ansprüche 10 bis 13,
dadurch **gekennzeichnet**, daß
sie mindestens eine fettlösliche Aktivsubstanz enthält, ausgewählt aus fettlöslichen Sonnenfilterstoffen, Substanzen zur Verbesserung des Zustands trockener oder gealterter Hautbereiche, aus Tocopherolen, Vitaminen E, F oder A und deren Estern, aus Retinoesäure, Antioxidantien, essentiellen Fettsäuren, Glycyrrhetinsäure, Keratolytika und Carotinoiden.

**16.** Zusammensetzung in Form einer wässrigen Dispersion lipidischer Kügelchen gemäß einem jeden der Ansprüche 10 bis 15,

dadurch **gekennzeichnet**, daß
sie 2 bis 70 Gew.% einer mit Wasser nicht mischbaren flüssigen Phase enthält, die aus Ölen, Kohlenwasserstoffen, halogenierten Kohlenwasserstoffen, Perfluortributylamin, Polysiloxanen, Estern organischer Säuren, Ethern und Polyethern ausgewählt ist.

**17.** Verfahren zur Herstellung einer Zusammensetzung zur kosmetischen oder dermopharmazeutischen Verwendung,
dadurch **gekennzeichnet**, daß
man 0,05 bis 20 und vorzugsweise 0,1 bis 10 Gew.% mindestens eines Ceramids, enthaltend eine Verbindung der Formel (I) :

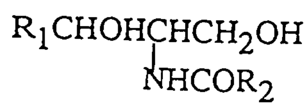 (I)

worin gilt:
- $R_1$ bedeutet einen $C_{11-21}$-Alkyl- oder -Alkenylrest,
- $R_2$ bedeutet einen linearen $C_{11-19}$-Kohlenwasserstoffrest, der eine oder mehrere, insbesondere eine oder zwei, ethylenisch ungesättigte Bindungen aufweist,
  oder enthaltend eine Mischung von Verbindungen der Formel (I) sowie der Formel:

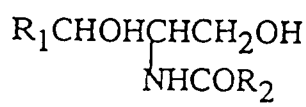 (Ia)

worin $R_1$ wie vorher definiert und $R_2$ ein linearer gesättigter $C_{11-19}$-Kohlenwasserstoffrest sind, wobei der Mengenanteil der Verbindungen der Formel (Ia) in der Mischung 35% nicht übersteigt, wobei das Ceramid in Form einer racemischen Mischung der erythro- und threo-Diastereoisomeren in erythro:threo-Mengenverhältnissen von 85:15 bis 60:40 vorliegt, in ein Trägermedium einbringt, das Hilsstoffe enthält, ausgewählt aus Fettkörpern, Lösungsmitteln, Wasser, Verdickungsmitteln, Emulgatoren, hydratisierenden Produkten, weichmachenden Mitteln, Sonnenfilterstoffen, Germiziden, Färbemitteln, Konservierungsstoffen, Parfüm-Produkten, Treibmitteln und aus oberflächenaktiven Mitteln.

**18.** Verfahren gemäß Anspruch 17 zur Herstellung einer Zusammensetzung zur kosmetischen oder dermopharmazeutichen Verwendung in Form einer wässrigen Dispersion lipidischer Kügelchen aus geordneten Molekularschichten, die eine eingekapselte wässrige Phase umschließen,
dadurch **gekennzeichnet**, daß
man zur Bildung dieser Schichten das oder die Ceramide zusammen mit mindestens einer weiteren lipidischen Verbindung verwendet.

**19.** Verfahren zur Herstellung einer wässrigen Dispersion lipidischer Kügelchen gemäß Anspruch 18,
dadurch **gekennzeichnet**, daß
man in die in die Kügelchen eingekapselte wässrige Phase mindestens eine wasserlösliche Substanz einbringt, die eine kosmetische und/oder pharmazeutische Wirksamkeit aufweist und aus Feuchtigkeitsmitteln, künstlichen Bräunungsmitteln, die gegebenenfalls zusammen mit Färbemitteln vorliegen, aus wasserlöslichen Sonnenfilterstoffen, Antiausdünnmitteln, Deodoriermitteln, zusammenziehenden Mitteln, Erfrischungsprodukten, tonischen Mitteln, Linderungsmitteln, Keratolytika, Enthaarungsmitteln, parfümierten Wässern, Extrakten von pflanzlichen Geweben, wasserlöslichen Färbemitteln, Mitteln gegen Haarausfall, antiseborrheischen Mitteln, oxidierenden Mitteln, reduzierenden Mitteln, Vitaminen, Hormonen, Enzymen, Impfstoffen, entzündungshemmenden Mitteln, Antibiotika, Bakteriziden sowie aus cytotoxischen und antitumoralen Mitteln ausgewählt ist.

**20.** Verfahren zur Herstellung einer wässrigen Dispersion lipidischer Kügelchen gemäß Anspruch 18, dadurch **gekennzeichnet**, daß
man in die geordneten Molekularschichten der lipidischen Kügelchen mindestens eine fettlösliche Aktivsubstanz einbringt, ausgewählt aus fettlöslichen Sonnenfilterstoffen, Substanzen zur Verbesserung des Zustands trockener oder gealterter Hautbereiche, aus Tocopherolen, Vitaminen E, F oder A und deren Estern, aus Retinoesäure, Antioxidantien, essentiellen Fettsäuren, Glycyrrhetinsäure, Keratolytika und Carotinoiden.